# EUROPÄISCHE PATENTSCHRIFT

(11) **EP 4 100 503 B1**
(45) Veröffentlichungstag und Bekanntmachung des Hinweises auf die Patenterteilung: **30.07.2025**
(21) Anmeldenummer: 21704200.1
(22) Anmeldetag: 02.02.2021
(51) Int. Cl.: C12M 1/00, A61L 2/28, C12M 1/12, C12Q 1/22, G01N 31/22

(54) **VERFAHREN UND SYSTEM ZUM AUTOMATISIERTEN KEIMMONITORING IN EINEM ISOLATOR**
METHOD AND SYSTEM FOR AN AUTOMATED MICROBIAL MONITORING PROCESS IN AN ISOLATOR
PROCÉDÉ ET SYSTÈME DE SURVEILLANCE MICROBIENNE AUTOMATISÉE DANS UN ISOLATEUR

(30) Priorität: 04.02.2020 DE 102020102758
(43) Veröffentlichungstag der Anmeldung: 14.12.2022
(73) Patentinhaber: GRONINGER & CO. GMBH, 74564 Crailsheim (DE)
(72) Erfinder: MERZ, Armin, 73479 Ellwangen (DE); ENGELHARD, Roland, 91589 Aurach-Weinberg (DE)
(74) Vertreter: Witte, Weller & Partner Patentanwälte mbB
(86) Internationale Anmeldenummer: PCT/EP2021/052446
(87) Internationale Veröffentlichungsnummer: WO 2021/156263

(56) Entgegenhaltungen:
- DE-A1- 102009 039 547
- US-A1- 2004 185 521
- US-A1- 2005 042 710

## Beschreibung

Die vorliegende Erfindung betrifft ein Verfahren, ein System und eine Computerprogramm zum automatisierten Keimmonitoring in einem Isolator.

Die vorliegende Erfindung beschäftigt sich in erster Linie mit aseptischen Isolatoren, die beispielsweise einen Füllbereich zum fluiden Befüllen von Objekten (z.B. Vials, Karpulen, Fläschchen, Spritzen und/oder dergleichen) mittels Füllnadeln aufweisen. Unter dem Begriff "Isolator" ist allgemein ein Behälter zu verstehen, der gegenüber dem umgebenden Arbeitsraum hermetisch und gasdicht abgeschlossen ist. Innerhalb eines Isolators kann eine definierte Atmosphäre zur Bearbeitung empfindlicher oder gefährlicher Produkte erzeugt werden.

In diesem Kontext werden Isolatoren üblicherweise in der biopharmazeutischen Prozesstechnik, beispielsweise als Teil einer Füllanlage mit mehreren Prozess- und Verarbeitungsstationen, verwendet, um eine hochreine oder sterile, sprich keimfreie Umgebung zu schaffen und eine Kontamination durch Keime, insbesondere Bakterien, Viren, Krankheitserreger und/oder dergleichen, zu vermeiden.

Pharmazeutische Füllanlagen stehen in der Regel in einer keimarmen Umgebung. Im Füllbereich innerhalb des Isolators muss eine keimfreie Umgebung vorliegen. Dieser Zustand wird überwacht, indem an den kritischen Stellen Keimsammler platziert werden. Keimsammler können beispielsweise Petrischalen sein, die einen Nährboden aufweisen. Wenn ein Keim auf den Nährboden kommt, findet beim anschließenden Ausbrüten ein Wachstum des Keims statt, wodurch eine Kontamination rückwirkend nachgewiesen werden kann. Ein derartiges Überwachen einer keimfreien Umgebung wird als "Keimmonitoring" oder "mikrobiologisches Monitoring" bezeichnet.

Zum Keimmonitoring eines Isolators werden im Stand der Technik verschieden Verfahren und Systeme vorgeschlagen. Zum einen werden beim passiven Keimmonitoring Keimsammler verwendet, die innerhalb des Isolators positioniert werden und an denen Umgebungsluft vorbeiströmt. Keime, die in der vorbeiströmenden Luft vorhanden sind, lassen sich auf dem Nährboden nieder. Diese Keimsammler werden insbesondere mittels Handschuheingriff vor Produktionsbeginn steril über Transferschleusen, beispielsweise Alpha-Beta-Ports, in den Isolator eingebracht und innerhalb des Isolators positioniert. Die Petrischalen können zudem einen Deckel aufweisen, der ebenfalls mittels Handschuheingriff abgenommen wird.

Da die Nährböden im Laufe der Zeit austrocknen, werden sie in der Regel nach maximal vier Stunden ausgetauscht. Dazu muss die Produktion unterbrochen werden. Mittels erneuten Handschuheingriffs wird der Deckel wieder auf die bis dahin verwendete Petrischale gelegt und die Petrischale zurück in den Port transferiert. Von außen wird die Petrischale dann aus dem Port entnommen und steril in einen Brutschrank transportiert. Vor erneutem Produktionsbeginn wird eine neue Petrischale mit Nährboden mittels Handschuheingriff eingebracht und der Deckel abgenommen.

Zum anderen werden beim aktiven Keimmonitoring die Keimsammler in ein Gehäuse eingesetzt, die innerhalb des Isolators angeordnet sind. Die Gehäuse saugen aktiv Luft an, die dann an den Keimsammlern vorbeiströmt. Das Gehäuse weist einen Deckel auf. Das Ein- und Ausschleusen von Keimsammlern erfolgt beim aktiven Keimmonitoring ähnlich wie beim passiven Keimmonitoring mittels Handschuheingriff. Zusätzlich muss beim aktiven Keimmonitoring noch der Deckel des Gehäuses entfernt werden, bevor die Keimsammler in das Gehäuse eingesetzt werden können.

Diese "manuelle" Handhabung mittels Handschuheingriff ist jedoch enorm zeitintensiv. Des Weiteren können Handschuhdurchführungen, welche in der Praxis aus Gummi- oder Kunststoff, insbesondere Butyl, bestehen, beim Greifen der Petrischalen oder der Deckel beschädigt werden. Handschuheingriffe stellen somit auch ein erhöhtes Kontaminations- und/oder Sicherheitsrisiko infolge von Leckagen dar. Zudem kann es durch manuellen Handschuheingriff zu einer Verschleppung von auf dem Nährboden angeordneten Keimen kommen.

Im Stand der Technik ist allgemein bekannt, dass Objekte, beispielsweise Petrischalen, innerhalb eines Isolators mittels Robotern gehandhabt werden können.

Beispielsweise zeigt die Druckschrift JP 2017 113836 A eine Hand für ein Zellkulturgefäss, die an einer Spitze eines Mehrgelenkroboters montierbar ist, um ein Zellkulturgefäss mit einem Deckel zu handhaben. Die Hand umfasst: einen Handstiel; ein Behälterhalteteil an einer Unterseite zum lösbaren Halten des Zellkulturgefässes; und ein Deckelhalteteil an einer Oberseite zum lösbaren Halten des Deckels. In der Hand für ein Zellkulturgefäss sind das Gefässhalteteil und das Deckelhalteteil derart am Handstiel angebracht, dass sie sich gegenseitig nähern und vertikal trennbar sind.

Des Weiteren zeigt die Druckschrift DE 10 2015 210 842 B3 eine Vorrichtung zum Greifen, Vereinzeln, Transportieren und/oder Ablegen von Petrischalen und ein Verfahren zu ihrem Betrieb. Die Vorrichtung weist eine Verfahreinheit auf, die von einer damit verbundenen elektronischen Steuereinheit gesteuert ist und mindestens einen Freiheitsgrad aufweist. An/in der Verfahreinheit ist ein Greifer angeordnet, der zwei horizontal ausgerichtete Rahmenelemente aufweist, die Petrischalen verschiedener Durchmesser entlang ihres Umfangs zumindest teilweise umgreifen und die mit einem Antrieb verbunden sind, der mit der elektronischen Steuereinheit verbunden ist, so dass die Rahmenelemente horizontal aufeinander zu und voneinander weg bewegbar sind. Dabei ist der Abstand der Rahmenelemente zueinander über die elektronische Steuereinheit einstellbar. An jedem Rahmenelemente ist jeweils ein Greifelement zum Erfassen und Halten von Petrischalen zwischen den Greifelementen der beiden Rahmenelemente angeordnet, wobei die Greifelemente vertikal beweglich gelagert und mit an den Rahmenelementen und Greifelementen befestigten Federn in eine Ursprungsposition rückholbar sind. An mindestens einem Greifelement ist ein Bauelement angeordnet, das sich gemeinsam mit dem Greifelement bewegt und mit einem zugeordneten Sensor zur Erfassung des Bauelements, der an einem Rahmenelement angeordnet und mit der elektronischen Steuereinheit verbunden ist, eine Detektion der Position des Bauelements erfolgt. An jedem Rahmenelement sind außerdem ausfahrbare Einheiten angeordnet, die mit der elektronischen Steuereinheit verbunden und von ihr gesteuert sind, und die mittels Hubmagneten, mit Federn zum Rückholen in eine Ursprungsposition, eine Ausstoßplatte vertikal nach unten bewegen, wobei die Ausstoßplatten beim Umgreifen einer Petrischale am Boden der jeweiligen Petrischale anliegen.

Die Druckschrift US 2004/0185521 A1 zeigt, um in einem ersten Isolator vor, während und nach dem Betrieb eines Abfüllsystems Mikroorganismen zu beproben, dass ein erster und ein zweiter Roboter, die in dem ersten Isolator bereitgestellt sind, ein Gerät zur Probenahme zu einer ersten und einer zweiten Probennahmeposition tragen, um natürlich herabfallende Mikroorganismen zu beproben, das Gerät zur Probenahme zu einer dritten Probennahmeposition tragen, um Mikroorganismen zu beproben, die um einen Füllstutzen herum schwimmen, und beproben Mikroorganismen, die an den Flächen des vorbestimmten Füllstutzens und einer Kappenzuführung an der vierten und fünften Probennahmeposition anhaften.

Die bekannten Systeme und Verfahren zum Keimmonitoring lassen noch Raum für Verbesserungen hinsichtlich Handhabung und Betriebssicherheit.

Vor diesem Hintergrund ist es eine Aufgabe der vorliegenden Erfindung, ein verbessertes Verfahren, ein verbessertes System und ein Computerprogramm bereitzustellen, das eine bessere Handhabung und Betriebssicherheit ermöglicht.

Gemäß einem ersten Aspekt wird ein Verfahren zum automatisierten Keimmonitoring in einem Isolator bereitgestellt. Der Isolator weist eine Transferschleuse auf. Das Verfahren weist die folgenden Schritte auf:
- erstes Bereitstellen mindestens eines Nährbodenträgerhalters an jeweils einer ersten Position innerhalb des Isolators;
- zweites Bereitstellen mindestens eines Nährbodenträgers innerhalb der Transferschleuse;
- erstes robotergestütztes Transferieren jeweils eines einzelnen Nährbodenträgers des mindestens einen Nährbodenträgers von der Transferschleuse zu einem freien Nährbodenträgerhalter des mindestens einen Nährbodenträgerhalters; und
- erstes robotergestütztes Anordnen des transferierten Nährbodenträgers in dem freien Nährbodenträgerhalter.

Gemäß einem zweiten Aspekt wird ein System zum automatisierten Keimmonitoring in einem Isolator bereitgestellt. Das System weist den Isolator, mindestens einen Nährbodenträgerhalter, einen in dem Isolator angeordneten Roboter und eine Steuerungseinrichtung auf, wobei der Isolator eine Transferschleuse aufweist, wobei in der Transferschleuse mindestens ein Nährbodenträger bereitgestellt ist, wobei der Nährbodenträgerhalter innerhalb des Isolators an einer ersten Position angeordnet ist, wobei der Roboter einen Endeffektor zum Handhaben eines Nährbodenträgers und eine Trägerstruktur zum Tragen des Endeffektors aufweist, wobei die Trägerstruktur dazu ausgebildet ist, den Endeffektor im Isolator zu bewegen, wobei der Endeffektor dazu ausgebildet ist, den Nährbodenträger zu greifen, wobei die Steuerungseinrichtung dazu eingerichtet ist, die folgenden Schritte auszuführen:
- erstes robotergestütztes Transferieren jeweils eines einzelnen Nährbodenträgers des mindestens einen Nährbodenträgers von der Transferschleuse zu einem freien Nährbodenträgerhalter des mindestens einen Nährbodenträgerhalters; und
- erstes robotergestütztes Anordnen des transferierten Nährbodenträgers in dem freien Nährbodenträgerhalter.

Gemäß einem dritten Aspekt wird ein Computerprogramm mit einem Programmcode bereitgestellt, der dazu ausgebildet ist, wenn er in der Steuerungseinrichtung des Systems nach dem zweiten Aspekt ausgeführt wird, die folgenden Schritte auszuführen:
- erstes robotergestütztes Transferieren jeweils eines einzelnen Nährbodenträgers des mindestens einen Nährbodenträgers von der Transferschleuse zu einem freien Nährbodenträgerhalter des mindestens einen Nährbodenträgerhalters; und
- erstes robotergestütztes Anordnen des transferierten Nährbodenträgers in dem freien Nährbodenträgerhalter.

Der Isolator ist vorzugsweise ein aseptischer Isolator. Ein aseptischer Isolator weist eine hochreine oder sterile, sprich keimfreie Umgebung auf. Der Isolator weist eine Transferschleuse auf. Eine derartige Transferschleuse kann vorliegend beispielsweise ein flexibler Sterilbeutel sein, der die in dem Sterilbeutel anordenbaren Gegenstände (z.B. Füllnadeln, Nadelträger, Objekte, etc.) vorsterilisiert über ein Schleusen- bzw. Schließsystem dem aseptischen Isolator, vorzugsweise zum Befüllen der Objekte, zuführt. In der Isolatortechnik sind derartige Sterilbeutel bekannt. Diese weisen in der Regel einen Adapter auf, mit dem der Sterilbeutel beispielsweise mit einem sogenannten Alpha- Port des aseptischen Isolators steril gekoppelt werden kann. Ein Alpha-Port kann eine Aussparung,z.B. ein Durchgangsloch und/oder eine Tür oder dergleichen, in einem Wandabschnitt des aseptischen Isolators sein. Der Sterilbeutel kann einen Beta-Port aufweisen, der z.B. als eine Tür oder dergleichen ausgebildet sein kann, die derart an dem Alpha-Port angebracht ist, dass der Alpha-Port und der Beta-Port gemeinsam geöffnet werden können.

Alternativ kann die Transferschleuse jedoch auch als starrer Transportbehälter ausgebildet sein, die nach dem vorgenannten Prinzip mit dem Alpha- Port des aseptischen Isolators koppelbar ist, um ein steriles Verbringen der darin anordenbaren Gegenstände in den aseptischen Isolator zu ermöglichen.

Unter dem Begriff "Nährbodenträger" ist vorliegend eine Vorrichtung zu verstehen, die dazu ausgebildet ist einen Nährboden zu tragen. Ein Nährbodenträger kann beispielsweise eine Petrischale oder eine Agar-Platte, in der der Nährboden angeordnet ist.

Unter dem Begriff "Nährbodenträgerhalter" ist vorliegend eine Vorrichtung zu verstehen, auf der ein Nährbodenträger abgestellt oder abgesetzt werden kann. Der Nährbodenträgerhalter kann dazu eine Auflagefläche aufweisen, auf der der Nährbodenträger abgestellt oder abgesetzt werden kann. Alternativ kann der Nährbodenträgerhalter auch eine Aufnahme aufweisen, in die der Nährbodenträger eingesetzt werden kann.

Unter dem Begriff "robotergestützt" ist zu verstehen, dass etwas mit Hilfe eines Roboters ausgeführt wird. Beispielsweise bedeutet "robotergestütztes Transferieren eines Nährbodenträgers", dass der Nährbodenträger mit Hilfe des Roboters transferiert wird. Dazu kann der Roboter beispielsweise eine bewegliche Trägerstruktur aufweisen, an deren Ende ein Endeffektor montiert ist, wobei der Endeffektor den Nährbodenträger zum Transferieren greifen kann.

Wie eingangs erwähnt, ist unter dem Begriff "Isolator" vorliegend ein Behälter zu verstehen, der gegenüber dem umgebenden Arbeitsraum hermetisch und gasdicht abgeschlossen ist. Es kann für den Isolator auch der Begriff Isolatorkammer verwendet werden. Innerhalb des Isolators kann eine definierte Atmosphäre zur Bearbeitung empfindlicher oder gefährlicher Produkte, insbesondere pharmazeutischer oder kosmetischer Produkte, erzeugt werden. Der Isolator kann ein aseptischer Isolator sein. Ein aseptischer Isolator kann beispielsweise ein Reinraum, Reinstraum oder dergleichen sein.

Ferner bezeichnet der Begriff "Transferschleuse" vorliegend eine Einrichtung zum Übergang zwischen zwei Bereichen mit vorzugsweise unterschiedlichen Eigenschaften, insbesondere einem Bereich innerhalb des aseptischen Isolators und einem Bereich außerhalb des aseptischen Isolators. In dem Kontext der vorliegenden Erfindung ist die Transferschleuse vorzugsweise derart ausgebildet, dass innerhalb der Transferschleuse ein aseptischer Zustand bereitgestellt ist oder wahlweise hergestellt werden kann. Dadurch kann sichergestellt werden, dass die zu transferierenden Gegenstände den bestehenden aseptischen Zustand innerhalb des aseptischen Isolators nicht beeinträchtigen bzw. zerstören. Derartige Verfahren zum Herstellen einer aseptischen Umgebung innerhalb der Transferschleuse sind in der Industrie bekannt.

Der Begriff "robotergestützt" bzw. "Roboter" ist vorliegend derart zu verstehen, dass die gekennzeichneten Verfahrensschritte mittels einer automatisierten Bewegungsapparatur jeglicher Art ausgeführt werden. Dabei kann es sich beispielsweise um eine Handlingseinheit, einen Manipulator, eine Kinematik oder dergleichen handeln, die den Roboter ausbildet. Ein "Roboter" meint eine Bewegungsapparatur, die zumindest eine, insbesondere gelenkige, Trägerstruktur aufweist, an deren Ende ein Roboter-Endeffektor angeordnet ist. Die Trägerstruktur ist dazu ausgebildet, den Roboter-Endeffektor in allen drei Raumrichtungen zu bewegen.

Bei der Bewegungsapparatur kann es sich beispielsweise um eine Konstruktion mit Mehrachs-Bewegungen jeglicher Art handeln. Beispielsweise kann eine derartige Konstruktion Zwei- bis Sechsachsbewegungen jeglicher Art aufweisen.

Zum ersten robotergestützten Transferieren kann der zu transferierende Nährbodenträger in oder an der Transferschleuse angeordnet sein. Unter "an der Transferschleuse" ist zu verstehen, dass der Nährbodenträger sich zumindest teilweise aus der Transferschleuse in den Isolator hinein erstreckt. In anderen Worten kann der Nährbodenträger zumindest teilweise oder vollständig innerhalb des Isolators, insbesondere an die Transferschleuse angrenzend oder benachbart zu der Transferschleuse, angeordnet sein. In dem Schritt des robotergestützten Transferierens des Nährbodenträgers von der Transferschleuse zu dem freien Nährbodenträger wird der Nährbodenträger somit robotergestützt von seiner Anordnung in oder an der Transferschleuse zu dem freien Nährbodenträgerhalter transferiert.

Durch das robotergestützte Transferieren eines Nährbodenträgers von der Transferschleuse zu einem Nährbodenträgerhalter innerhalb des Isolators und durch das robotergestützte anordnen des Nährbodenträgers auf dem Nährbodenträgerhalter wird ein automatisiertes Keimmonitoring ermöglicht. Dadurch sind die Handhabung und die Betriebssicherheit beim Keimmonitoring verbessert.

Die eingangs gestellte Aufgabe wird somit vollumfänglich gelöst.

In einer ersten Ausgestaltung weist das Verfahren vor dem Schritt des ersten robotergestützten Transferierens den folgenden Schritt auf:
- erstes robotergestütztes Entnehmen des zu transferierenden Nährbodenträgers aus der Transferschleuse.

Unter "robotergestütztem Entnehmen eines Nährbodenträgers" versteht man, dass mittels eines Roboters der Nährbodenträger aus der Transferschleuse entnommen wird. Zum Entnehmen kann der Roboter beispielsweise den Nährbodenträger greifen. Auf diese Weise kann der Nährbodenträger auf einfache Weise in den Isolator eingebracht werden.

In einer weiteren Ausgestaltung weist jeder Nährbodenträger eine Schale mit Nährboden und einen Deckel auf, der auf einer Öffnung der Schale aufgesetzt ist, wobei das Verfahren des Weiteren die folgenden Schritte aufweist:
- drittes Bereitstellen mindestens eines Deckelhalters zur Ablage des Deckels des transferierten Nährbodenträgers, wobei der mindestens eine Deckelhalter an jeweils einer zweiten Position innerhalb des Isolators bereitgestellt wird.

Unter dem Begriff " Deckelhalter" ist vorliegend eine Vorrichtung zu verstehen, auf der ein Deckel eines Nährbodenträgers abgelegt werden kann. Der Deckelhalter kann dazu eine Auflagefläche aufweisen, auf der der Deckel abgelegt werden kann. Alternativ kann der Deckelhalter auch eine Aufnahme aufweisen, in die der Deckel eingesetzt werden kann. Auf diese Weise wird ermöglicht, dass, selbst wenn der Nährbodenträger einen Deckel aufweist, das automatisierte Keimmonitoring in dem Isolator mittels eines Roboters ausgeführt werden kann. Ohne Deckelhalter müsste der Deckel während des gesamten Keimmonitorings bis zu dem Zeitpunkt, an dem der Deckel wieder aufgesetzt werden darf, mittels des Roboters gehalten werden, wodurch der Roboter in dieser Zeit keine weiteren Operationen durchführen kann.

In einer weiteren Ausgestaltung weist das Verfahren des Weiteren die folgenden Schritte auf:
- robotergestütztes Abnehmen des Deckels von der Schale des transferierten Nährbodenträgers;
- zweites robotergestütztes Transferieren des Deckels von dem Nährbodenträgerhalter zu einem freien Deckelhalter des mindestens einen Deckelhalters; und
- robotergestütztes Ablegen des Deckels auf dem freien Deckelhalter.

Unter "robotergestütztem Abnehmen eines Deckel" versteht man, dass mittels eines Roboters der Deckel von dem Nährbodenträger abgenommen wird. Zum Abnehmen kann der Roboter beispielsweise den Deckel greifen und anheben. Unter "robotergestütztem Ablegen eines Deckel" versteht man, dass mittels eines Roboters der Deckel auf dem Deckelhalter abgelegt wird. Zum Ablegen kann der Roboter beispielsweise den Deckel auf dem Deckelhalter aufsetzten und freigeben. Auf diese Weise kann der Deckel möglichst einfach von dem Nährbodenträger ohne Handschuheingriff abgenommen werden. Zudem wird auch hierdurch ermöglicht, dass das automatisierte Keimmonitoring in dem Isolator mittels eines Roboters ausgeführt werden kann.

In einer weiteren Ausgestaltung weist das Verfahren des Weiteren die folgenden Schritte auf, nachdem der transferierte Nährbodenträger eine vordefinierte Zeitspanne in dem entsprechenden Nährbodenträgerhalter verbracht hat:
- robotergestütztes Entnehmen des Deckels von dem Deckelhalter;
- drittes robotergestütztes Transferieren des Deckels von dem Deckelhalter zu dem entsprechenden Nährbodenträgerhalter; und
- robotergestütztes Aufsetzen des Deckels auf die Schale des Nährbodenträgers.

Unter "robotergestütztem Entnehmen eines Deckel" versteht man, dass mittels eines Roboters der Deckel von dem Deckelhalter entnommen wird. Zum Entnehmen kann der Roboter beispielsweise den Deckel greifen und anheben. Unter "robotergestütztem Aufsetzen eines Deckel" versteht man, dass mittels eines Roboters der Deckel auf der Schale des Nährbodenträgers aufgesetzt wird. Zum Aufsetzen kann der Roboter beispielsweise den Deckel auf der Schale aufsetzten und freigeben. Auf diese Weise kann der Deckel möglichst einfach auf den Nährbodenträger ohne Handschuheingriff aufgesetzt werden.

In einer weiteren Ausgestaltung weist das Verfahren des Weiteren die folgenden Schritte auf, nachdem der transferierte Nährbodenträger eine vordefinierte Zeitspanne in dem entsprechenden Nährbodenträgerhalter verbracht hat:
- zweites robotergestütztes Entnehmen des Nährbodenträgers aus dem Nährbodenträgerhalter;
- viertes robotergestütztes Transferieren des Nährbodenträgers von dem Nährbodenträgerhalter zu der Transferschleuse; und
- zweites robotergestütztes Anordnen des Nährbodenträgers in der Transferschleuse.

Auf diese Weise kann der Nährbodenträger möglichst einfach ohne Handschuheingriff durch die Transferschleuse aus dem Isolator ausgeschleust werden, um den Nährbodenträger dann in einem Brutschrank zu geben. Die vordefinierte Zeitspanne kann beispielsweise vier Stunden betragen.

In einer weiteren Ausgestaltung wird in dem Schritt des ersten Bereitstellens eine Keimmonitoringeinrichtung mit zumindest einem Nährbodenträgerhalter bereitgestellt.

Die Keimmonitoringeinrichtung kann beispielsweise zum aktiven Keimmonitoring verwendet werden, wobei aktiv Umgebungsluft angesaugt wird, um diese an einem in dem Nährbodenträgerhalter angeordneten Nährbodenträger vorbeiströmen zu lassen. Auf diese Weise kann auch das aktive Keimmonitoring automatisiert betrieben werden.

In einer weiteren Ausgestaltung weist die Keimmonitoringeinrichtung ein Gehäuse auf, in dem der zumindest eine Nährbodenträgerhalter angeordnet ist, wobei das Gehäuse einen Gehäusedeckelaufweist, wobei das Verfahren des Weiteren die folgenden Schritte aufweist:
- viertes Bereitstellen eines Gehäusedeckelhalters innerhalb des Isolators an einer dritten Position;
- robotergestütztes Abnehmen des Gehäusedeckels; und
- robotergestütztes Ablegen des Gehäusedeckels auf dem Gehäusedeckelhalter.

Unter dem Begriff "Gehäusedeckelhalter" ist vorliegend eine Vorrichtung zu verstehen, auf der der Gehäusedeckel abgelegt werden kann. Der Gehäusedeckelhalter kann dazu eine Auflagefläche aufweisen, auf der der Gehäusedeckel abgelegt werden kann. Alternativ kann der Gehäusedeckelhalter auch eine Aufnahme aufweisen, in die der Gehäusedeckel eingesetzt werden kann. Auf diese Weise wird ermöglicht, dass das automatisierte Keimmonitoring in dem Isolator mittels nur eines Roboters ausgeführt werden kann.

In einer weiteren Ausgestaltung wird in dem Schritt des zweiten Bereitstellens eine Mehrzahl von Nährbodenträgern in der Transferschleuse bereitgestellt, wobei in dem Schritt des dritten Bereitstellens eine Lagereinrichtung mit einer Mehrzahl von Haltern an der zweiten Position bereitgestellt wird, die jeweils als Nährbodenträgerhalter oder Deckelhalter dienen können.

Auf diese Weise können die Mehrzahl von Nährbodenträgern in der Lagereinrichtung gelagert werden. Die Halter dienen dabei als Nährbodenträgerhalter. Zum Keimmonitoring kann dann immer jeweils ein Nährbodenträger aus dem Lager entnommen werden und in den Nährbodenträgerhalter der Keimmonitoringeinrichtung eingesetzt werden. Dadurch wird in der Lagereinrichtung ein Halter frei, in den dann der Deckel des in den Nährbodenträgerhalter der Keimmonitoringeinrichtung eingesetzten Nährbodenträgers abgelegt werden kann. Dieser Halter dient dann als Deckelhalter. Auf diese Weise kann die Anzahl der Ein- und Ausschleusvorgänge durch die Transferschleuse reduziert werden, wodurch das Kontaminationsrisiko vermindert wird.

In einer weiteren Ausgestaltung wird in dem Schritt des ersten Bereitstellens die Mehrzahl von Nährbodenträgern in einer Nährbodenträgerhalteeinrichtung in der Transferschleuse bereitgestellt.

Die Nährbodenträgerhalteeinrichtung kann mehrere Nähbodenträgerhalter für die Nährbodenträger aufweisen. Auf diese Weise können eine Mehrzahl von Nährbodenträgern möglichst einfach und geordnet in der Transferschleuse bereitgestellt werden. Dadurch wird auch das Entnehmen der einzelnen Nährbodenträger aus der Transferschleuse verbessert. Nach der vordefinierten Zeitspanne können die Nährbodenträger wieder in die Nährbodenträgerhalteeinrichtung zurückgesetzt werden. Dadurch können mehrere Nährbodenträger gleichzeitigt ein- und ausgeschleust werden, wodurch der Austausch von Nährböden schneller und effizienter erfolgen kann.

In einer weiteren Ausgestaltung wird in dem Schritt des zweiten Bereitstellens jeder Nährbodenträger in einem weiteren Nährbodenträgerhalter bereitgestellt, wobei jeder weitere Nährbodenträgerhalter in der Transferschleuse angeordnet wird, insbesondere wobei die Nährbodenträgerhalteeinrichtung jeden weiteren Nährbodenträgerhalter aufweist.

Auch hierdurch kann jeder Nährbodenträgern möglichst einfach und geordnet in der Transferschleuse bereitgestellt werden. Dadurch wird auch das Entnehmen jedes einzelnen Nährbodenträgers aus der Transferschleuse verbessert. Nach der vordefinierten Zeitspanne kann jeder Nährbodenträger wieder in den entsprechenden Nährbodenträgerhalter zurückgesetzt werden.

In einer weiteren Ausgestaltung kann sich jeder weitere Nährbodenträgerhalter zumindest teilweise aus der Transferschleuse heraus in den Isolator erstrecken kann, insbesondere wobei der Schritt des ersten robotergestützten Transferierens von der Transferschleuse zu dem freien Nährbodenträgerhalter derart erfolgt, dass sich der jeweilige weitere Nährbodenträgerhalter zumindest teilweise aus der Transferschleuse heraus in den Isolator erstreckt und der entsprechende Nährbodenträger robotergestützt von dem jeweiligen weiteren Nährbodenträgerhalter zu dem freien Nährbodenträgerhalter innerhalb des Isolators transferiert wird.

Auf diese Weise kann jeder Nährbodenträger besser und einfacher aus dem entsprechenden weiteren Nährbodenträgerhalter entnommen werden, um ihn dann robotergestützt zu dem freien Nährbodenträgerhalter innerhalb des Isolators zu transferieren.

In einer weiteren Ausgestaltung ist in dem Isolator ein Roboter angeordnet, wobei der Roboter einen Endeffektor zum Handhaben eines Nährbodenträgers und eine Trägerstruktur zum Tragen des Endeffektors aufweist, wobei die Trägerstruktur dazu ausgebildet ist, den Endeffektor im Isolator zu bewegen, wobei der Endeffektor dazu ausgebildet ist, den Nährbodenträger zu greifen.

Der Endeffektor kann des Weiteren dazu ausgebildet ein, den Deckel des Nährbodenträgers zu greifen. Der Endeffektor kann des Weiteren dazu ausgebildet ein, den Gehäusedeckel des Gehäuses zu greifen. Die Trägerstruktur kann beispielsweise gelenkig, insbesondere mehrgelenkig ausgebildet sein, wobei die Trägerstruktur mittels einer oder mehrerer Antriebseinrichtungen bewegt wird. Der Endeffektor kann an einem Ende der Trägerstruktur drehbar gelagert sein. Auf diese Weise kann der Nährbodenträger oder der Deckel oder der Gehäusedeckel auf einfache Weise mit Hilfe des Roboters in dem Isolator transferiert werden.

In einer weiteren Ausgestaltung ist die Anzahl der Nährbodenträger, die in der Transferschleuse bereitgestellt werden, gleich eins, zwei, drei, vier, fünf, sechs, sieben, acht, neun, zehn, elf oder zwölf.

Mit ein bis vier Nährbodenträgern kann auf effiziente Art und Weise das automatisierte Keimmonitoring in einem Isolator betrieben werden.

In einer weiteren Ausgestaltung ist die Anzahl der Nährbodenträgerhalter, die in dem Isolator bereitgestellt werden, gleich oder größer der Anzahl der Nährbodenträger, die in der Transferschleuse bereitgestellt werden.

Auf diese Weise wird ermöglicht, dass für jeden Nährbodenträger, der in die Transferschleuse eingebracht wird, ein freier Nährbodenträgerhalter vorhanden ist.

In einer weiteren Ausgestaltung ist die Anzahl der Deckelhalter, die in dem Isolator bereitgestellt werden, gleich oder größer der Anzahl der Nährbodenträger, die in der Transferschleuse bereitgestellt werden

Auf diese Weise wird ermöglicht, dass für jeden Nährbodenträger, der in die Transferschleuse eingebracht wird, ein freier Deckelhalter vorhanden ist, auf den der Deckel des Nährbodenträgers abgelegt werden kann. Die Nährbodenträger können nach und nach in die Monitoringposition gebracht werden. In diesem Fall ist immer nur ein Deckelhalter in Gebrauch. Es kann dann nur ein einziger Deckelhalter notwendig und vorhanden. Es können aber auch mehrere Monitoringpositionen aus einem Vorrat bedient. Dann werden mehr Deckelhalter benötigt.

In einer weiteren Ausgestaltung ist die Anzahl der Halter der Lagereinrichtung gleich oder größer der Anzahl der Nährbodenträger, die in der Transferschleuse bereitgestellt werden.

Auf diese Weise wird ermöglicht, dass genügend Nährbodenträgerhalter und Deckelhalter vorhanden sind, wenn immer ein geöffneter Nährbodenträger in dem Nährbodenträgerhalter der Keimmonitoringeinrichtung angeordnet wird.

In einer weiteren Ausgestaltung weist der Endeffektor eine Aufnahme zum Aufnehmen des Nährbodenträger auf, die zwischen einer Aufnahmestellung, in der der Nährbodenträger aufgenommen werden kann, und einer Greifstellung, in der der Nährbodenträger gegriffen werden kann, verlagerbar ist, insbesondere wobei jeder Nährbodenträger zum Transferieren jeweils mittels des Endeffektors gegriffen und mittels des Roboters im Isolator bewegt werden kann.

In anderen Worten weist der Endeffektor zum Greifen eine Aufnahme auf, die zwischen einer Aufnahmestellung und einer Greifstellung verlagerbar ist. In der Aufnahmestellung ist die Aufnahme so weit geöffnet, dass der Nährbodenträger oder der Deckel oder der Gehäusedeckel in die Aufnahme eingebracht werden kann. In der Greifstellung ist die Aufnahme so weit geschlossen, dass der Nährbodenträger oder der Deckel oder der Gehäusedeckel gegriffen wird. Auf diese Weise kann der Nährbodenträger oder der Deckel oder der Gehäusedeckel auf einfache Weise mit Hilfe des Roboters in dem Isolator transferiert werden.

In einer weiteren Ausgestaltung weist das System eine Keimmonitoringeinrichtung mit zumindest einem Nährbodenträgerhaltern aufweist.

Die Keimmonitoringeinrichtung kann beispielsweise zum aktiven Keimmonitoring verwendet werden, wobei aktiv Umgebungsluft angesaugt wird, um diese an einem in dem Nährbodenträgerhalter angeordneten Nährbodenträger vorbeiströmen zu lassen. Auf diese Weise kann auch das aktive Keimmonitoring automatisiert betrieben werden.

In einer weiteren Ausgestaltung weist die Keimmonitoringeinrichtung ein Gehäuse auf, in der zumindest eine Nährbodenträgerhalter angeordnet ist, wobei das Gehäuse einen Gehäusedeckel aufweist, wobei der Gehäusedeckel mittels des Roboters abnehmbar ist, wobei das System einen Gehäusedeckelhalter zur Ablage des Gehäusedeckels aufweist, wobei der Gehäusedeckelhalters innerhalb des Isolators an einer dritten Position angeordnet ist.

Der Gehäusedeckelhalter kann dazu eine Auflagefläche aufweisen, auf der der Gehäusedeckel abgelegt werden kann. Alternativ kann der Gehäusedeckelhalter auch eine Aufnahme aufweisen, in die der Gehäusedeckel eingesetzt werden kann. Auf diese wird ermöglicht, dass das automatisierte Keimmonitoring in dem Isolator mittels nur eines Roboters ausgeführt werden kann.

In einer weiteren Ausgestaltung weist jeder Nährbodenträger eine Schale mit Nährboden und einen Deckel auf, der auf einer Öffnung der Schale aufgesetzt ist, wobei der Deckel mittels des Roboters abnehmbar ist, wobei das System des Weiteren mindestens einen Deckelhalter zur Ablage des Deckels aufweist, wobei der zumindest eine Deckelhalter innerhalb des Isolator an jeweils einer zweiten Position angeordnet ist, wobei.

Mittels eines Deckelhalters wird ermöglicht, dass, selbst wenn der Nährbodenträger einen Deckel aufweist, das automatisierte Keimmonitoring in dem Isolator mittels eines Roboters ausgeführt werden kann. Insbesondere kann das System eine Deckelhaltereinrichtung mit einer Mehrzahl von Deckelhaltern aufweisen. Auf diese Weise können eine Mehrzahl von Deckeln möglichst einfach und geordnet auf den jeweiligen Deckelhaltern abgelegt werden. Dadurch wird das Lagern der Deckel während des Keimmonitorings ermöglicht.

In einer weiteren Ausgestaltung weist das System des Weiteren eine Lagereinrichtung mit einer Mehrzahl von Haltern auf, wobei die Lagereinrichtung an der zweiten Position angeordnet ist, wobei die Halter jeweils als Nährbodenträgerhalter oder Deckelhalter dienen können.

Auf diese Weise können die Mehrzahl von Nährbodenträgern in der Lagereinrichtung gelagert werden. Die Halter dienen dabei als Nährbodenträgerhalter. Zum Keimmonitoring kann dann immer jeweils ein Nährbodenträger aus dem Lager entnommen werden und in den Nährbodenträgerhalter der Keimmonitoringeinrichtung eingesetzt werden. Dadurch wird in der Lagereinrichtung ein Halter frei, in den dann der Deckel des in den Nährbodenträgerhalter der Keimmonitoringeinrichtung eingesetzten Nährbodenträgers abgelegt werden kann. Dieser Halter dient dann als Deckelhalter. Auf diese Weise kann die Anzahl der Ein- und Ausschleusvorgänge durch die Transferschleuse reduziert werden, wodurch das Kontaminationsrisiko vermindert wird.

Es versteht sich, dass die voranstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsformen der Erfindung sind in der Zeichnung dargestellt und werden in der nachfolgenden Beschreiung näher erläutert. Es zeigen:
- Fig. 1: eine schematische Darstellung einer ersten Ausführungsform eines Systems zum automatisierten Keimmonitoring in einem Isolator;
- Fig. 2A, 2B: eine schematische Darstellung einer ersten Ausführungsform eines Verfahrens zum automatisierten Keimmonitoring in einem Isolator;
- Fig. 3: eine schematische Darstellung einer zweiten Ausführungsform eines Systems zum automatisierten Keimmonitoring in einem Isolator;
- Fig. 4: eine isometrische Ansicht des Systems aus Fig. 3;
- Fig. 5: eine isometrische Ansicht eines Endeffektors mit Nährbodenträger;
- Fig. 6: eine isometrische Ansicht einer Transferschleuse mit darin angeordneten Nährbodenträgern;
- Fig. 7: eine schematische Darstellung einer zweiten Ausführungsform eines Verfahrens zum automatisierten Keimmonitoring in einem Isolator;
- Fig. 8A: eine Draufsicht auf das System aus Fig. 4 beim Abnehmen des Gehäusedeckels von dem Gehäuse der Keimmonitoringeinrichtung;
- Fig. 8B: eine Draufsicht auf das System aus Fig. 4 beim Ablegen des Gehäusedeckels auf dem Gehäusedeckelhalter;
- Fig. 9A: eine Draufsicht auf das System aus Fig. 4 beim Entnehmen des Nährbodenträgers aus der Transferschleuse;
- Fig. 9B: eine Draufsicht auf das System aus Fig. 4 beim Anordnen des Nährbodenträgers auf dem Nährbodenträgerhalter der Keimmonitoringeinrichtung;
- Fig. 10A: eine Draufsicht auf das System aus Fig. 4 beim Abnehmen des Deckels von dem Nährbodenträger;
- Fig. 10B: eine Draufsicht auf das System aus Fig. 4 beim Ablegen des Deckels auf einem Halter, der als Deckelhalter dient;
- Fig. 11A: eine Draufsicht auf das System aus Fig. 4 beim Aufnehmen des Gehäusedeckels von dem Gehäusedeckelhalter;
- Fig. 11B: eine Draufsicht auf das System aus Fig. 4 beim Aufsetzen des Gehäusedeckels auf dem Gehäuse der Keimmonitoringeinrichtung; und
- Fig. 12A, 12B, 12C: eine schematische Darstellung einer dritten Ausführungsform eines Verfahrens zum automatisierten Keimmonitoring in einem Isolator.

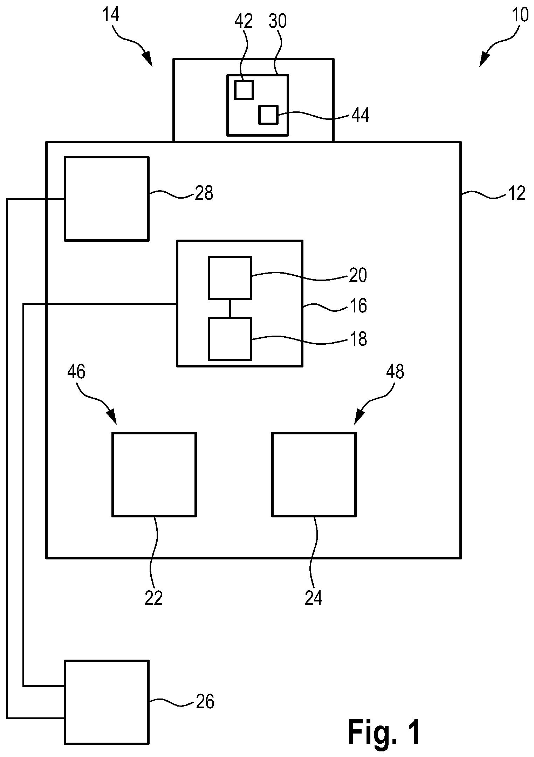

Fig. 1 zeigt eine erste Ausführungsform eines Systems 10 zum automatisierten Keimmonitoring in einem Isolator 12. Das System 10 weist den Isolator 12 auf. Der Isolator 12 weist eine Transferschleuse 14 auf. Die Transferschleuse 14 grenzt an den Isolator 12 an. In der Transferschleuse 14 kann ein Nährbodenträger 30 bereitgestellt werden. Der Nährbodenträger 30 weist eine Schale 42 mit Nährboden und einen Deckel 44 auf. Der Deckel 44 kann auf die Schale 42 aufgesetzt werden, um die Schale zu verschließen, oder von der Schale 42 abgenommen werden, um die Schale 42 zu öffnen. Im geöffneten Zustand kommt Umgebungsluft in Kontakt mit dem Nährboden. In der Transferschleuse 14 kann auch eine Mehrzahl von Nährbodenträgern 30 bereitgestellt werden. Beispielsweise kann in der Transferschleuse 14 eine Nährbodenträgerhalteeinrichtung bereitgestellt werden, die eine Mehrzahl von Nährbodenträgerhaltern aufweist, in denen die Nährbodenträger 30 der Mehrzahl von Nährbodenträgern angeordnet sind.

Das System 10 weist des Weiteren einen Roboter 16 auf. Der Roboter 16 ist in dem Isolator 12 angeordnet. Der Roboter 16 weist eine Trägerstruktur 18 und einen Endeffektor 20 auf. Der Endeffektor 20 ist dazu ausgebildet, einen Nährbodenträger oder zu handhaben. Insbesondere ist der Endeffektor 20 dazu ausgebildet, den Nährbodenträger 30 zu greifen. Die Trägerstruktur 18 ist dazu ausgebildet, den Endeffektor 20 zu tragen. Insbesondere ist die Trägerstruktur 18 dazu ausgebildet, den Endeffektor 20 im Isolator 12 zu bewegen. Dazu kann die Trägerstruktur 18 beispielsweise gelenkig, insbesondere mehrgelenkig, ausgebildet sein. Des Weiteren kann der Endeffektor 20 an einem Ende der Trägerstruktur 18 angeordnet und insbesondere drehbar gelagert sein. Der Roboter 16 kann des Weiteren eine oder mehrere Antriebseinrichtungen aufweisen, die dazu ausgebildet sind die Trägerstruktur 18 zu bewegen und den Endeffektor 20 an dem Ende der Trägerstruktur 18 zu drehen, wodurch der Endeffektor 20 im Isolator bewegt und ausgerichtet werden kann.

Der Endeffektor 20 kann beispielsweise eine Aufnahme zum Aufnehmen des Nährbodenträgers 30 aufweisen. Die Aufnahme kann zwischen einer Aufnahmestellung, in der der Nährbodenträger 30 oder der Deckel 44 aufgenommen werden kann, und einer Greifstellung, in der der Nährbodenträger 30 oder der Deckel 44 gegriffen werden kann, verlagerbar sein. Um die Aufnahme zwischen der Aufnahmestellung und der Greifstellung zu verlagern, kann der Endeffektor 20 eine Antriebseinrichtung aufweisen. Zum Transferieren kann der Nährbodenträger 30 mittels des Endeffektors 20 gegriffen und mittels des Roboters 16 im Isolator bewegt werden kann.

Das System 10 weist des Weiteren einen Nährbodenträgerhalter 22 auf. Der Nährbodenträgerhalter 22 ist an einer ersten Position 46 innerhalb des Isolators 12 bereitgestellt. Der Nährbodenträgerhalter 22 ist derart ausgebildet, dass ein Nährbodenträger, beispielsweise den Nährbodenträger 30, auf dem Nährbodenträgerhalter 22 abgestellt bzw. abgesetzt werden kann. Dazu kann der Nährbodenträgerhalter 22 beispielsweise eine Auflagefläche aufweisen, auf der der Nährbodenträger 30 abgestellt oder abgesetzt werden kann. Das System kann eine Mehrzahl von Nährbodenträgerhaltern 22 aufweisen, die jeweils an, insbesondere unterschiedlichen, ersten Positionen 46 in dem Isolator 12 bereitgestellt sind. Vorzugsweise ist die Anzahl der Nährbodenträgerhalter 22 gleich oder größer der Anzahl der bereitgestellten Nährbodenträger 30.

Das System 10 weist des Weiteren einen Deckelhalter 24 auf. Der Deckelhalter 24 ist an einer zweiten Position 48 innerhalb des Isolators 12 bereitgestellt. Der Deckelhalter 24 ist derart ausgebildet, dass ein Deckel eines Nährbodenträgers, beispielsweise der Deckel 44 des Nährbodenträgers 30, auf dem Deckelhalter 24 abgelegt werden kann. Dazu kann der Deckelhalter 24 beispielsweise eine Auflagefläche aufweisen, auf der Deckel 44 des Nährbodenträgers 30 abgelegt werden kann. Das System kann eine Mehrzahl von Deckelhaltern 24 aufweisen, die jeweils an, insbesondere unterschiedlichen, zweiten Positionen 48 in dem Isolator 12 bereitgestellt sind. Vorzugsweise ist die Anzahl der Deckelhalter 24 gleich oder größer der Anzahl der bereitgestellten Nährbodenträger 30.

Das System 10 weist des Weiteren eine Steuerungseinrichtung 26 auf. Die Steuerungseinrichtung 26 ist dazu ausgebildet, den Roboter 16 zu steuern. Die Steuerungseinrichtung 26 kann den Roboter derart steuern, dass der Endeffektor 20 im Isolator 12 bewegen und ausgerichtet wird und dass mittels des Endeffektors Nährbodenhalter und Deckel entnommen oder aufgenommen und abgesetzt oder abgelegt werden können. Die Steuerungseinrichtung 26 kann dazu beispielsweise Steuersignale an den Roboter 16 senden. Insbesondere kann die Steuerungseinrichtung 26 Steuersignale an die Antriebseinrichtungen des Roboters 16 senden, um die den Endeffektor im Raum zu bewegen und auszurichten. Des Weiteren kann die Steuerungseinrichtung 26 Steuersignale an die Antriebseinrichtung des Endeffektors senden, um die Aufnahme des Endeffektors 20 zwischen der Aufnahmestellung und der Greifstellung zu verlagern.

Das System 10 kann des Weiteren eine Sensoreinrichtung 28 aufweisen. Die Sensoreinrichtung 28 ist dazu ausgebildet, die Position und Ausrichtung des Endeffektors und des zu transferierenden Objekts, beispielsweise des Nährbodenträgers 30 oder des Deckels 44, zu bestimmen. Dazu kann die Sensoreinrichtung 28 beispielsweise optische Sensoren aufweisen. Die Sensoreinrichtung 28 kann dazu ausgebildet sein, Sensorsignale an die Steuerungseinrichtung 26 zu senden. Die Sensorsignale können beispielweise Informationen über die Position und Ausrichtung des Endeffektors und des zu transferierenden Objekts enthalten. Die Steuerungseinrichtung 26 kann des Weiteren dazu ausgebildet sein, den Roboter 16 auf Basis der empfangenen Sensorsignale zu steuern.

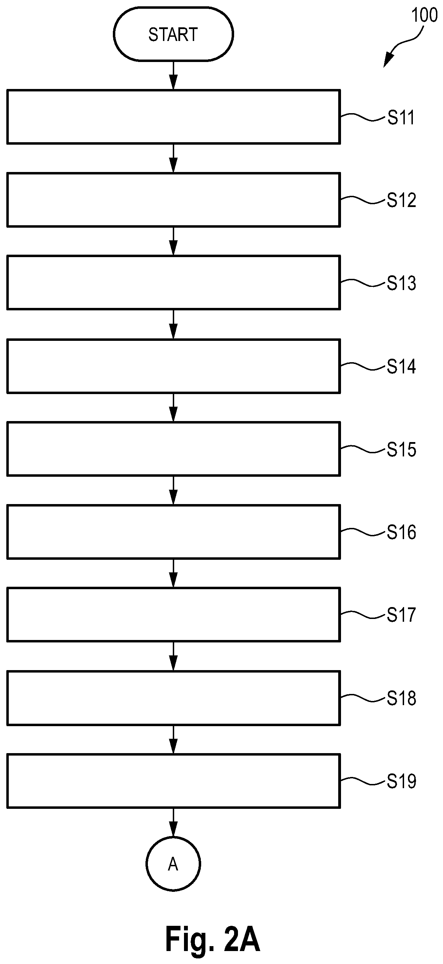

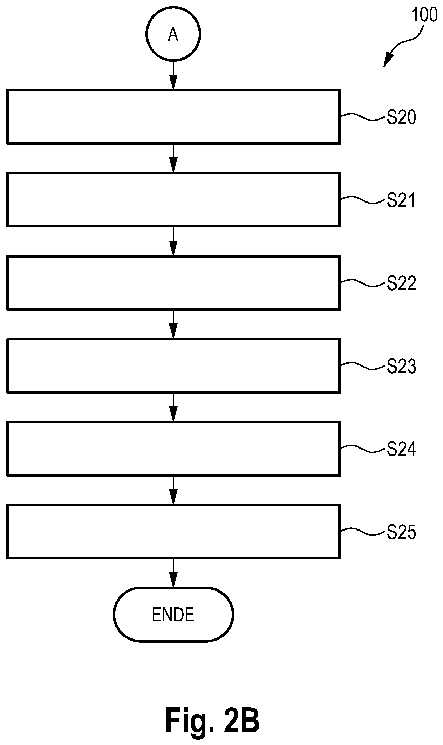

Fig. 2 zeigt eine erste Ausführungsform eines Verfahrens 100 zum automatisierten Keimmonitoring in einem Isolator 12. Das Verfahren kann beispielsweise mittels des Systems 10 aus Fig. 1 durchgeführt werden. Insbesondere kann die Steuerungseinrichtung 26 dazu ausgebildet sein die Schritte S14 bis S25 durchzuführen.

In einem ersten Schritt S11 des Verfahrens 100 wird mindestens ein Nährbodenträgerhalter 22 an jeweils einer ersten Position 46 innerhalb des Isolators 12 bereitgestellt.

In einem weiteren Schritt S12 des Verfahrens 100 wird mindestens ein Deckelhalter 24 zur Ablage des Deckels 44 eines transferierten Nährbodenträgers 30 an jeweils einer zweiten Position 48 innerhalb des Isolators 12 bereitgestellt.

Die Schritte S11 bis S12 können in einer beliebigen Reihenfolge durchgeführt werden.

In einem weiteren Schritt S13 des Verfahrens 100 wird mindestens ein Nährbodenträger 30 innerhalb der Transferschleuse 14 bereitgestellt.

In einem weiteren Schritt S14 des Verfahrens 100 wird jeweils ein einzelner Nährbodenträger 30 des mindestens einen Nährbodenträgers 30 robotergestützt aus der Transferschleuse 14 entnommen. Dazu kann der Roboter 16 beispielsweise derart gesteuert werden, dass der Endeffektor 20 zu dem zu transferierenden Nährbodenträger 30 bewegt wird und der Endeffektor 20 den zu transferierenden Nährbodenträger 30 greift.

In einem weiteren Schritt S15 des Verfahrens 100 wird der entnommene Nährbodenträger 30 robotergestützt von der Transferschleuse 14 zu einem freien Nährbodenträgerhalter 22 des mindestens einen Nährbodenträgerhalters 22 transferiert. Dazu kann beispielsweise der Roboter 16 beispielsweise derart gesteuert werden, dass der Endeffektor 20 von der Transferschleuse 14 zu dem freien Nährbodenträgerhalter 22 bewegt wird.

In einem weiteren Schritt S16 des Verfahrens 100 wird der transferierte Nährbodenträger 30 robotergestützt in dem feien Nährbodenträgerhalter 22 angeordnet. Dabei wird der Nährbodenträger 30 insbesondere auf einer Auflagefläche des Nährbodenträgerhalters 22 abgesetzt. Dazu kann der Roboter beispielsweise derart gesteuert werden, dass der Endeffektor 20 den zu transferierenden Nährbodenträger 30 auf der Auflagefläche aufsetzt und freigibt.

In einem weiteren Schritt S17 des Verfahrens 100 wird der Deckel 44 des transferierten Nährbodenträgers 30 robotergestützt von der Schale 42 abgenommen. Dazu kann der Roboter 16 beispielsweise derart gesteuert werden, dass der Endeffektor 18 den Deckel 44 greift und anhebt.

In einem weiteren Schritt S18 des Verfahrens 100 wird der Deckel 44 robotergestützt von dem Nährbodenträgerhalter 22 zu einem freien Deckelhalter 24 des mindestens einen Deckelhalters 24 transferiert. Dazu kann der Roboter 16 beispielsweise derart gesteuert werden, dass der Endeffektor 18 den gegriffen Deckel 44 von dem Nährbodenträgerhalter 22 zu dem freien Deckelhalter 24 bewegt.

In einem weiteren Schritt S19 des Verfahrens 100 wird der transferierte Deckel 44 robotergestützt auf dem freien Deckelhalter 24 abgelegt. Dazu kann der Roboter 16 beispielsweise derart gesteuert werden, dass der Endeffektor 18 den transferierten Deckel 44 auf dem Deckelhalter 24 ablegt, insbesondere absetzt und freigibt.

Wenn eine Mehrzahl von Nährbodenträgern 30 in der Transferschleuse 14 bereitgestellt sind, können die Schritte S14 bis S19 für jeden Nährbodenträger 30 wiederholt werden.

Jede Schale 42 des mindestens einen Nährbodenträgers 30 verbleibt dann eine vordefinierte Zeitspanne in dem jeweiligen Nährbodenträgerhalter 22 in geöffneten Zustand, wodurch das Keimmonitoring in dieser Zeitspanne ausgeführt wird. Die vordefinierte Zeitspanne kann beispielsweise 4 Stunden betragen.

In einem weiteren Schritt S20 des Verfahrens 100 wird, nachdem der transferierte Nährbodenträger 30 die vordefinierte Zeitspanne in dem entsprechenden Nährbodenträgerhalter 22 verbracht hat, der Deckel **44** des Nährbodenträgers 30 robotergestützt von dem Deckelhalter 24 aufgenommen. Dazu kann der Roboter 16 beispielsweise derart gesteuert werden, dass der Endeffektor 18 den Deckel **44** greift und anhebt.

In einem weiteren Schritt S21 des Verfahrens 100 wird der Deckel **44** robotergestützt von dem Deckelhalter 24 zu dem Nährbodenträgerhalter 22 transferiert, auf dem die Schale 42 des Nährbodenträgers 30 angeordnet ist. Dazu kann der Roboter 16 beispielsweise derart gesteuert werden, dass der Endeffektor 18 den gegriffen Deckel 44 von dem Deckelhalter 46 zu dem Nährbodenträgerhalter 22 bewegt.

In einem weiteren Schritt S22 des Verfahrens 100 wird der Deckel 44 robotergestützt auf der Schale 42 des Nährbodenträgers 30 aufgesetzt. Dazu kann der Roboter 16 beispielsweise derart gesteuert werden, dass der Endeffektor 18 den Deckel 44 auf der Schale 42 aufsetzt und freigibt.

In einem weiteren Schritt S23 des Verfahrens 100 wird der Nährbodenträger 30 robotergestützt aus dem Nährbodenträgerhalter 22 entnommen. Dazu kann der Roboter 16 beispielsweise derart gesteuert werden, dass der Endeffektor 18 den geschlossenen Nährbodenträger 30 greift.

In einem weiteren Schritt S24 des Verfahrens 100 wird der entnommene Nährbodenträger 30 robotergestützt von dem Nährbodenträgerhalter 22 zu der Transferschleuse 14 transferiert. Dazu kann der Roboter 16 beispielsweise derart gesteuert werden, dass der Endeffektor 18 den Nährbodenträger 30 von dem Nährbodenträgerhalter 22 zu der der Transferschleuse 14 bewegt.

In einem weiteren Schritt S25 des Verfahrens 100 wird der transferierte Nährbodenträger 30 robotergestützt in der Transferschleuse 14 angeordnet. Dazu kann der Roboter 16 beispielsweise derart gesteuert werden, dass der Endeffektor 18 den Nährbodenträger 30 in der Transferschleuse 14 ablegt und freigibt.

Wenn eine Mehrzahl von Nährbodenträgern 30 verwendet werden, können die Schritte S20 bis S25 für jeden Nährbodenträger 30 wiederholt werden.

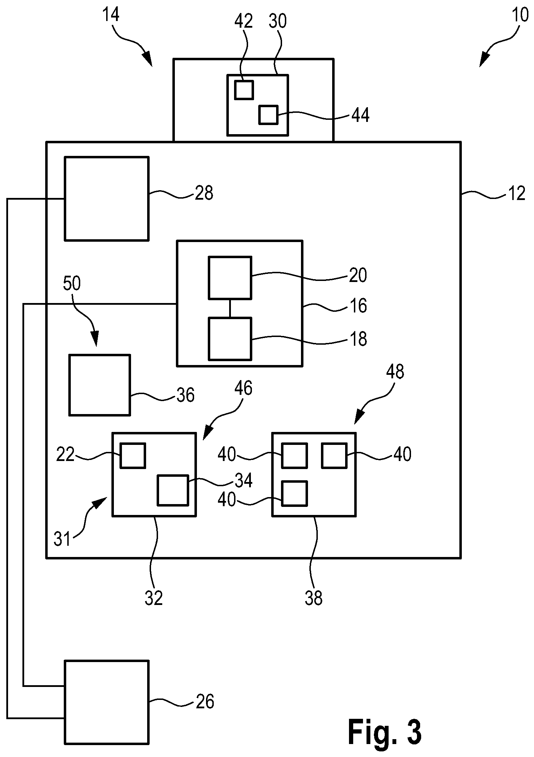

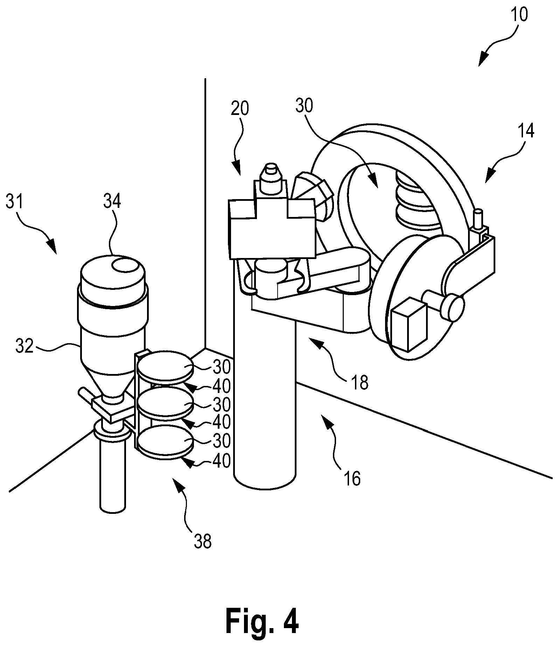

Die Figuren 3 und 4 zeigen eine zweite Ausführungsform eines Systems 10 zum automatisierten Keimmonitoring in einem Isolator 12. Das System 10 entspricht im Wesentlichen dem System 10 der ersten Ausführungsform aus Fig. 1. Gleiche Elemente sind mit den gleichen Bezugszeichen gekennzeichnet und werden nochmal erläutert.

Das System 10 der zweiten Ausführungsform weist des Weiteren eine Keimmonitoringeinrichtung 31 auf. Die Keimmonitoringeinrichtung 31 weist ein Gehäuse 32 und eine Gehäusedeckel 34 auf. Die Keimmonitoringeinrichtung 31 weist den Nährbodenträgerhalter 22 auf. Der Nährbodenträgerhalter 22 in ist dem Gehäuse 32 angeordnet. In dem Gehäuse 32 kann auch eine Mehrzahl von Nährbodenträgerhaltern 22 angeordnet sein.

Das System 10 der zweiten Ausführungsform weist des Weiteren einen Gehäusedeckelhalter 36 auf. Der Gehäusedeckelhalter 36 ist innerhalb des Isolators an einer dritten Position 50 bereitgestellt. Der Gehäusedeckelhalter 36 ist derart ausgebildet, dass der Gehäusedeckel 34 auf dem Gehäusedeckelhalter 36 abgelegt werden kann. Dazu kann der Gehäusedeckelhalter 36 beispielsweise eine Auflagefläche aufweisen, auf der Gehäusedeckel 34 abgelegt werden kann.

Das System 10 der zweiten Ausführungsform weist des Weiteren eine Lagereinrichtung 38 auf. Die Lagereinrichtung 38 weist mindestens einen Halter 40 auf. Jeder Halter 40 dient jeweils als Nährbodenträgerhalter oder als Deckelhalter. Insbesondere ist jeder Halter 40 derart ausgebildet, dass sowohl ein gesamter Nährbodenträger 30 als auch ein Deckel 44 auf dem Halter 40 abgelegt werden kann. Die Lagereinrichtung 38 kann auch eine Mehrzahl von Haltern 40 aufweisen. Insbesondere kann die Lagereinrichtung 38 drei Haltern 40 aufweisen. Die Lagereinrichtung 38 kann an der Keimmonitoringeinrichtung 31 montiert sein. In Fig. 4 ist zudem exemplarisch dargestellt, wie drei Nährbodenträger 30 in den Haltern 40 angeordnet sind, wobei die Haltern 40 als Nährbodenträgerhalter dienen.

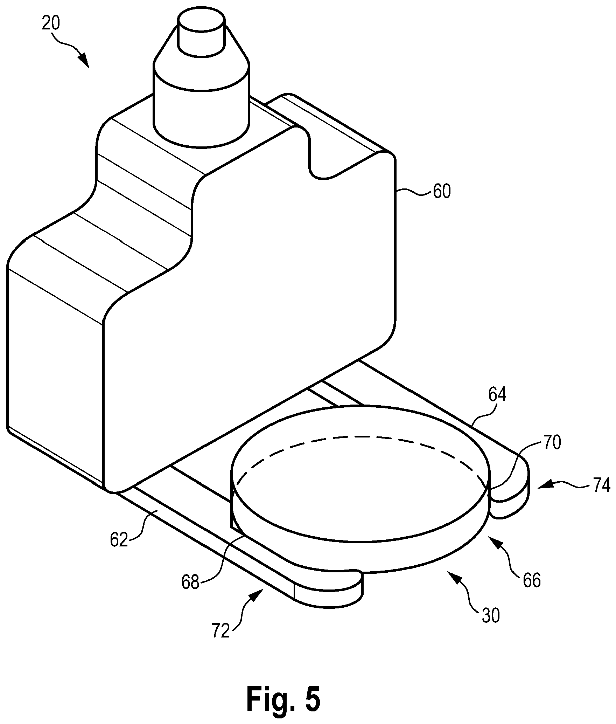

In Fig. 5 ist der Endeffektor 20 des Roboters 16 des Systems 10 der zweiten Ausführungsform im Detail dargestellt.

Der Endeffektor 20 weist eine Aufnahme 66 zum Aufnehmen eines Nährbodenträgers 30 oder eines Deckels 44 auf. Die Aufnahme 66 ist zwischen einer Aufnahmestellung, in der der Nährbodenträger 30 oder der Deckel 44 aufgenommen werden kann, und einer Greifstellung, in der der Nährbodenträger 30 oder der Deckel 44 gegriffen werden kann, verlagerbar. Dazu weist der Endeffektor 20 einen Grundkörper 60, ein erstes Verlagerungselement 62 und ein zweites Verlagerungselement 64 auf. Das erste Verlagerungselement 62 und das zweite Verlagerungselement 64 sind an einer Unterseite des dem Grundkörper 60 jeweils drehbar oder verschiebbar gelagert. Die Verlagerungselemente 62, 64 erstrecken sich parallel zueinander in eine Erstreckungsrichtung von dem Grundkörper 60 weg. An einem dem Grundkörper abgewandten Ende 72, 74 weist jedes Verlagerungselement 62, 64 jeweils einen Greifabschnitt 68, 70 auf. Die Aufnahme 66 ist zwischen den Verlagerungselementen 62, 64 angeordnet. Die Aufnahme 66 weist die Greifabschnitte 68 und 70 auf, wobei die Greifabschnitte 68, 70 auf gegenüberliegenden Seiten der Aufnahme 66 angeordnet sind.

Zum Verlagern der Aufnahme 66 werden die Verlagerungselemente 62, 64 derart verlagert, dass die Greifabschnitte zum Verlagern in die Greifstellung aufeinander zu bewegt werden und zum verlagern in die Aufnahmestellung voneinander weg bewegt werden. Die Verlagerungselemente 62, 64 können dazu entweder rotatorisch oder translatorisch verlagerbar sein. Um die Aufnahme 66 zwischen der Aufnahmestellung und der Greifstellung zu verlagern, kann der Endeffektor 20 eine Antriebseinrichtung aufweisen, die dazu ausgebildet ist, die Verlagerungselemente 62, 64 entsprechend translatorisch oder rotatorisch zu verlagern.

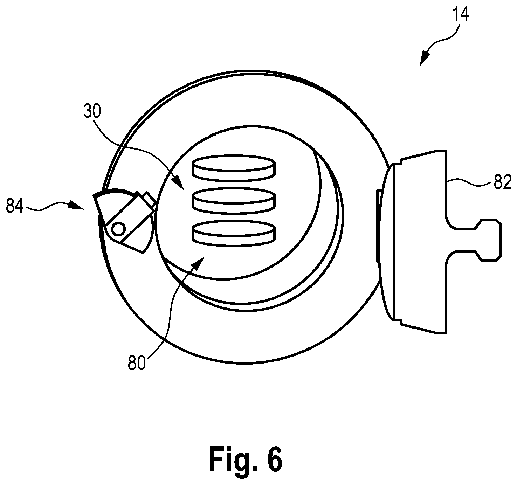

In Fig. 6 ist die Transferschleuse 14 des Isolators 12 des Systems 10 der zweiten Ausführungsform im Detail dargestellt.

Die Transferschleuse 14 weist einen Schleuseninnenraum 80 und eine Tür 82 auf. In dem Schleuseninnenraum können die Nährbodenträger 30 bereitgestellt werden. Die Tür 82 ist zwischen einer offenen Stellung, in der der Schleuseninnenraum 80 mit dem Innenraum des Isolators 12 verbunden ist, und eine geschlossenen Stellung, in der der Schleuseninnenraum 80 vom Innenraum des Isolators 12 getrennt ist, verlagerbar. Die Transferschleuse 14 weist des Weiteren ein Verriegelungselement 84 auf, mittels dem die Tür in der verschlossenen Stellung verriegelt werden kann. In Fig. 6 ist die Tür in der offenen Stellung dargestellt.

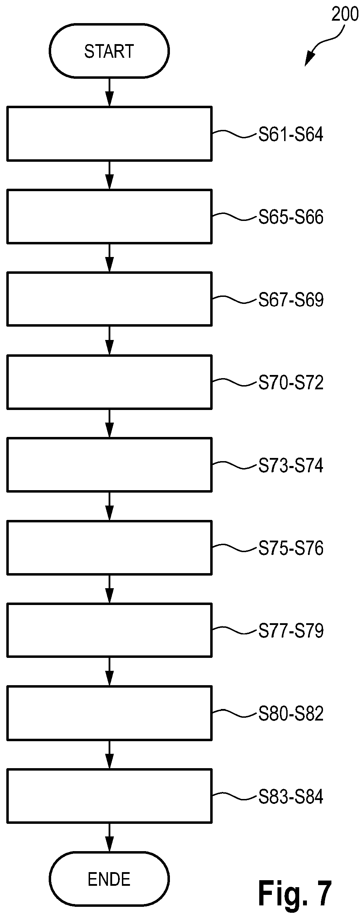

Fig. 7 zeigt eine zweite Ausführungsform eines Verfahrens 200 zum automatisierten Keimmonitoring in einem Isolator 12. Das Verfahren kann beispielsweise mittels des Systems 10 aus den Fig. 3 und 4 durchgeführt werden. Insbesondere kann die Steuerungseinrichtung 26 dazu ausgebildet sein, die Schritte S65 bis S84 durchzuführen.

In einem ersten Schritt S61 des Verfahrens 200 wird die Keimmonitoringeinrichtung 31 mit mindestens einem Nährbodenträgerhalter 22 an einer ersten Position 46 innerhalb des Isolators 12 bereitgestellt.

In einem weiteren Schritt S62 des Verfahrens 200 wird die Lagereinrichtung 38 mit mindestens einem Halter 40 an einer zweiten Position 48 innerhalb des Isolators 12 bereitgestellt, wobei der Halter 40 als Deckelhalter 24 zur Ablage des Deckels 44 eines transferierten Nährbodenträgers 30 dient.

In einem weiteren Schritt S63 des Verfahrens 200 wird ein Gehäusedeckelhalter 36 zur Ablage des Gehäusedeckels 34 an einer dritten Position 50 innerhalb des Isolators 12 bereitgestellt.

Die Schritte S61 bis S63 können in einer beliebigen Reihenfolge durchgeführt werden.

In weiteren Schritt S64 des Verfahrens 200 wird mindestens ein Nährbodenträger 30 innerhalb der Transferschleuse 14 bereitgestellt.

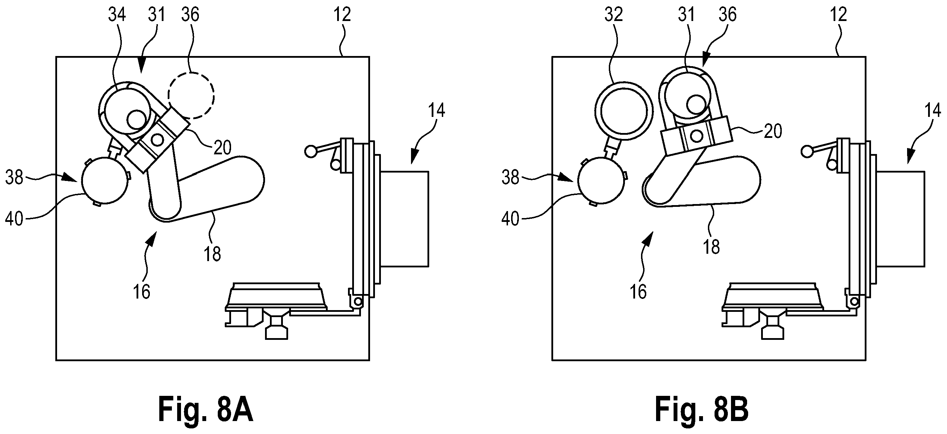

In einem weiteren Schritt S65 des Verfahrens 200 wird der Gehäusedeckel 34 robotergestützt von dem Gehäuse 32 der Keimmonitoringeinrichtung 31 abgenommen. Dazu kann der Roboter 16 beispielsweise derart gesteuert werden, dass der Endeffektor 18 den Gehäusedeckel 34 greift und anhebt. Dies ist exemplarisch in Fig. 8A dargestellt.

In einem weiteren Schritt S66 des Verfahrens 200 wird der Gehäusedeckel 34 robotergestützt auf dem Gehäusedeckelhalter 36 abgelegt. Dazu kann der Roboter 16 beispielsweise derart gesteuert werden, dass der Endeffektor 18 den Gehäusedeckel 34 auf dem Gehäusedeckelhalter 36 ablegt, insbesondere absetzt und freigibt. Dies ist exemplarisch in Fig. 8B dargestellt.

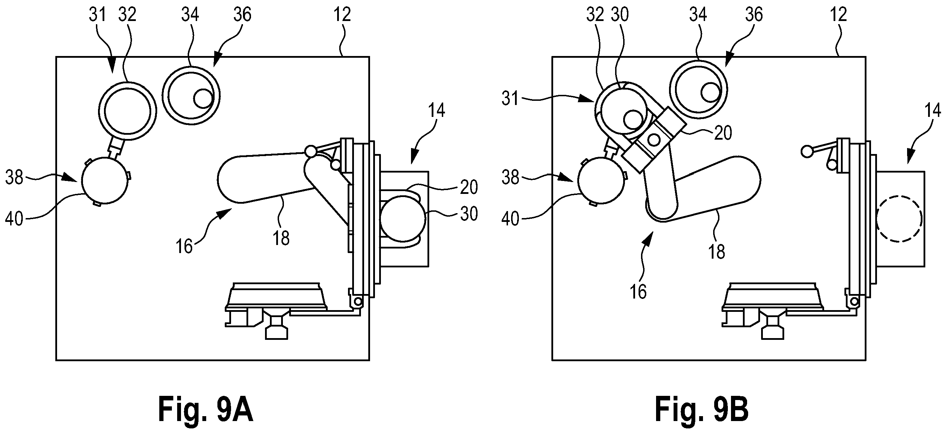

In einem weiteren Schritt S67 des Verfahrens 200 wird jeweils ein einzelner Nährbodenträger 30 des mindestens einen Nährbodenträgers 30 robotergestützt aus der Transferschleuse 14 entnommen. Dazu kann der Roboter 16 beispielsweise derart gesteuert werden, dass der Endeffektor 20 zu dem zu transferierenden Nährbodenträger 30 bewegt wird und der Endeffektor 20 den zu transferierenden Nährbodenträger 30 greift. Dies ist exemplarisch in Fig. 9A dargestellt.

In einem weiteren Schritt S68 des Verfahrens 200 wird der entnommene Nährbodenträger 30 robotergestützt von der Transferschleuse 14 zu einem freien Nährbodenträgerhalter 22 des mindestens einen Nährbodenträgerhalters 22 in dem Gehäuse 32 der Keimmonitoringeinrichtung 31 transferiert. Dazu kann beispielsweise der Roboter 16 beispielsweise derart gesteuert werden, dass der Endeffektor 20 von der Transferschleuse 14 zu dem freien Nährbodenträgerhalter 22 bewegt wird.

In einem weiteren Schritt S69 des Verfahrens 200 wird der transferierte Nährbodenträger 30 robotergestützt in dem feien Nährbodenträgerhalter 22 in dem Gehäuse 32 der Keimmonitoringeinrichtung 31 angeordnet. Dabei wird der Nährbodenträger 30 insbesondere auf einer Auflagefläche des Nährbodenträgerhalters 22 abgesetzt. Dazu kann der Roboter beispielsweise derart gesteuert werden, dass der Endeffektor 20 den zu transferierenden Nährbodenträger 30 auf der Auflagefläche aufsetzt und freigibt. Dies ist exemplarisch in Fig. 9B dargestellt.

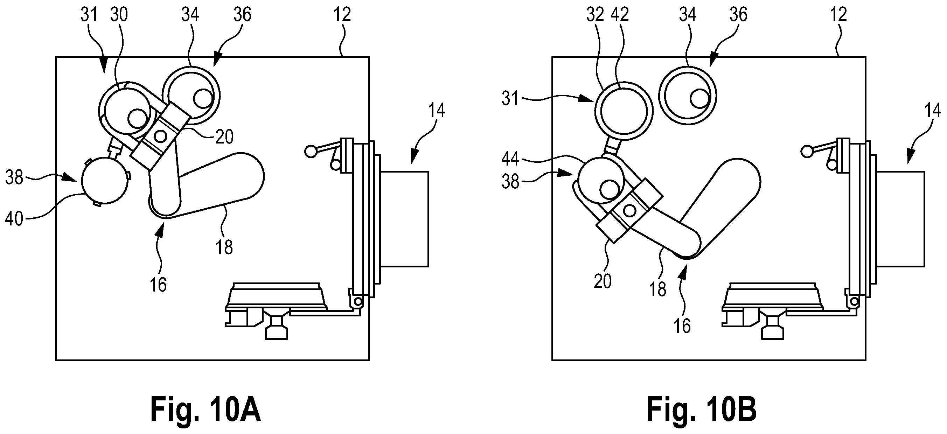

In einem weiteren Schritt S70 des Verfahrens 200 wird der Deckel 44 des transferierten Nährbodenträgers 30 robotergestützt von der Schale 42 abgenommen. Dazu kann der Roboter 16 beispielsweise derart gesteuert werden, dass der Endeffektor 18 den Deckel 44 greift und anhebt. Dies ist in Fig. 10A dargestellt.

In einem weiteren Schritt S71 des Verfahrens 200 wird der Deckel 44 robotergestützt von dem Nährbodenträgerhalter 22 zu einem freien Halter 40 der Lagereinrichtung 38 transferiert, wobei der Halter 40 als Deckelhalter 24 dient. Dazu kann der Roboter 16 beispielsweise derart gesteuert werden, dass der Endeffektor 18 den gegriffen Deckel 44 von dem Nährbodenträgerhalter 22 zu dem freien Halter 40 bewegt.

In einem weiteren Schritt S72 des Verfahrens 200 wird der transferierte Deckel 44 robotergestützt auf dem freien Halter 40, der als Deckelhalter 24 dient, abgelegt. Dazu kann der Roboter 16 beispielsweise derart gesteuert werden, dass der Endeffektor 18 den transferierten Deckel 44 auf dem Halter 40 ablegt, insbesondere absetzt und freigibt. Dies ist exemplarisch in Fig. 10B dargestellt.

Wenn eine Mehrzahl von Nährbodenträgern 30 in der Transferschleuse 14 bereitgestellt sind, können die Schritte S67 bis S72 für jeden Nährbodenträger 30 wiederholt werden.

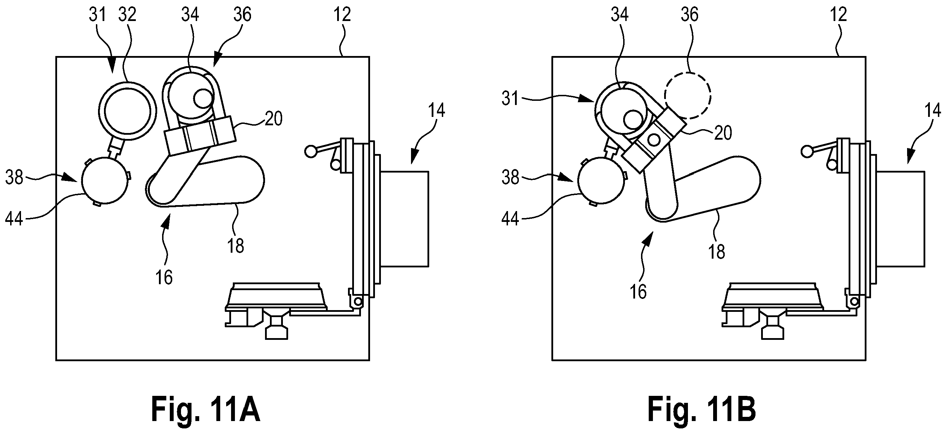

In einem weiteren Schritt S73 des Verfahrens 200 wird der Gehäusedeckel 34 robotergestützt von dem Gehäusedeckelhalter 36 aufgenommen. Dazu kann der Roboter 16 beispielsweise derart gesteuert werden, dass der Endeffektor 18 den Gehäusedeckel 34 greift und anhebt. Dies ist exemplarisch in Fig. 11A dargestellt.

In einem weiteren Schritt S74 des Verfahrens 200 wird der Gehäusedeckel 34 robotergestützt auf das Gehäuse 32 der Keimmonitoringeinrichtung 31 aufgesetzt. Dazu kann der Roboter 16 beispielsweise derart gesteuert werden, dass der Endeffektor 18 den Gehäusedeckel 34 auf dem Gehäuse 32 der Keimmonitoringeinrichtung 31 aufsetzt und freigibt. Dies ist exemplarisch in Fig. 11B dargestellt.

Jede Schale 42 des mindestens einen Nährbodenträgers 30 verbleibt dann eine vordefinierte Zeitspanne in dem jeweiligen Nährbodenträgerhalter 22 in geöffneten Zustand in der Keimmonitoringeinrichtung, wodurch das Keimmonitoring in dieser Zeitspanne ausgeführt wird. Die vordefinierte Zeitspanne kann beispielsweise 4 Stunden betragen.

In einem weiteren Schritt S75 des Verfahrens 200 wird, nachdem der transferierte Nährbodenträger 30 die vordefinierte Zeitspanne in dem entsprechenden Nährbodenträgerhalter 22 der Keimmonitoringeinrichtung 31 verbracht hat, der Gehäusedeckel 34 robotergestützt von dem Gehäuse 32 der Keimmonitoringeinrichtung 31 abgenommen. Dazu kann der Roboter 16 beispielsweise derart gesteuert werden, dass der Endeffektor 18 den Gehäusedeckel 34 greift und anhebt.

In einem weiteren Schritt S76 des Verfahrens 200 wird der Gehäusedeckel 34 robotergestützt auf dem Gehäusedeckelhalter 36 abgelegt. Dazu kann der Roboter 16 beispielsweise derart gesteuert werden, dass der Endeffektor 18 den Gehäusedeckel 34 auf dem Gehäusedeckelhalter 36 ablegt, insbesondere absetzt und freigibt.

In einem weiteren Schritt S77 des Verfahrens 200 wird der Deckel 44 des Nährbodenträgers 30 robotergestützt von dem Halter 40, der als Deckelhalter 24 dient, aufgenommen. Dazu kann der Roboter 16 beispielsweise derart gesteuert werden, dass der Endeffektor 18 den Deckel 44 greift und anhebt.

In einem weiteren Schritt S78 des Verfahrens 200 wird der Deckel 44 robotergestützt von dem Halter 40, der als Deckelhalter 24 dient, zu dem Nährbodenträgerhalter 22 in dem Gehäuse 32 der Keimmonitoringeinrichtung 31 transferiert, auf dem die Schale 42 des Nährbodenträgers 30 angeordnet ist. Dazu kann der Roboter 16 beispielsweise derart gesteuert werden, dass der Endeffektor 18 den gegriffen Deckel 44 von dem Halter 40, der als Deckelhalter 24 dient, zu dem Nährbodenträgerhalter 22 bewegt.

In einem weiteren Schritt S79 des Verfahrens 200 wird der Deckel 44 robotergestützt auf der Schale 42 des Nährbodenträgers 30 aufgesetzt. Dazu kann der Roboter 16 beispielsweise derart gesteuert werden, dass der Endeffektor 18 den Deckel 44 auf der Schale 42 aufsetzt und freigibt.

In einem weiteren Schritt S80 des Verfahrens 200 wird der Nährbodenträger 30 robotergestützt aus dem Nährbodenträgerhalter 22 aus dem Gehäuse 32 der Keimmonitoringeinrichtung 31 entnommen. Dazu kann der Roboter 16 beispielsweise derart gesteuert werden, dass der Endeffektor 18 den geschlossenen Nährbodenträger 30 greift.

In einem weiteren Schritt S81 des Verfahrens 200 wird der entnommene Nährbodenträger 30 robotergestützt von dem Nährbodenträgerhalter 22 in dem Gehäuse 32 der Keimmonitoringeinrichtung 31 zu der Transferschleuse 14 transferiert. Dazu kann der Roboter 16 beispielsweise derart gesteuert werden, dass der Endeffektor 18 den Nährbodenträger 30 von dem Nährbodenträgerhalter 22 zu der der Transferschleuse 14 bewegt.

In einem weiteren Schritt S82 des Verfahrens 200 wird der transferierte Nährbodenträger 30 robotergestützt in der Transferschleuse 14 angeordnet. Dazu kann der Roboter 16 beispielsweise derart gesteuert werden, dass der Endeffektor 18 den Nährbodenträger 30 in der Transferschleuse 14 ablegt und freigibt.

Wenn eine Mehrzahl von Nährbodenträgern 30 verwendet werden, können die Schritte S77 bis S82 für jeden Nährbodenträger 30 wiederholt werden.

In einem weiteren Schritt S83 des Verfahrens 200 wird der Gehäusedeckel 34 robotergestützt auf dem Gehäusedeckelhalter 36 abgelegt. Dazu kann der Roboter 16 beispielsweise derart gesteuert werden, dass der Endeffektor 18 den Gehäusedeckel 34 greift und anhebt.

In einem weiteren Schritt S84 des Verfahrens 200 wird der Gehäusedeckel 34 robotergestützt auf das Gehäuse 32 der Keimmonitoringeinrichtung 31 aufgesetzt. Dazu kann der Roboter 16 beispielsweise derart gesteuert werden, dass der Endeffektor 18 den Gehäusedeckel 34 auf dem Gehäuse 32 der Keimmonitoringeinrichtung 31 aufsetzt und freigibt.

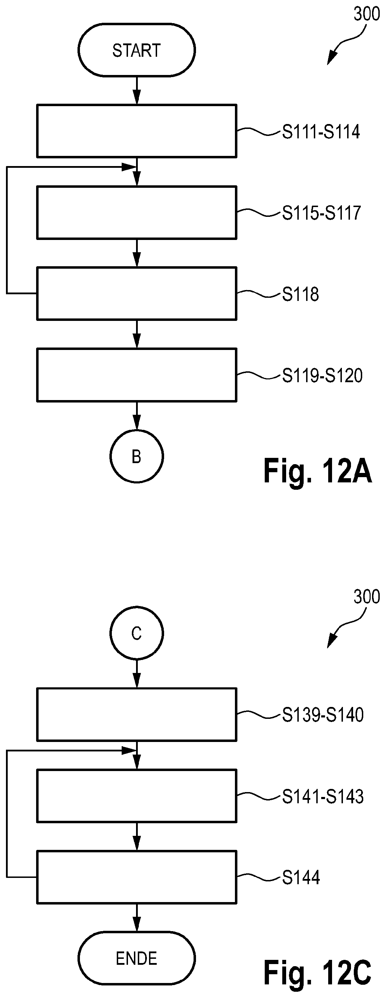

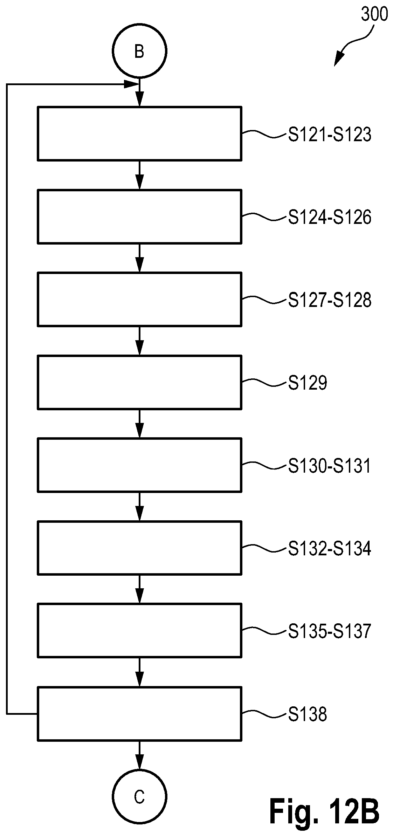

Die Figuren 12A, 12B und 12C zeigen eine dritte Ausführungsform eines Verfahrens 300 zum automatisierten Keimmonitoring in einem Isolator 12. Das Verfahren kann beispielsweise mittels des Systems 10 aus den Fig. 3 und 4 durchgeführt werden. Insbesondere kann die Steuerungseinrichtung 26 dazu ausgebildet sein die Schritte S115 bis S144 durchzuführen.

Die Schritte S111 bis S114 entsprechen den Schritten S61 bis S64 des Verfahrens 200 aus Fig. 7.

In einem weiteren Schritt S115 des Verfahrens 300 wird jeweils ein einzelner Nährbodenträger 30 des mindestens einen Nährbodenträgers 30 robotergestützt aus der Transferschleuse 14 entnommen. Dazu kann der Roboter 16 beispielsweise derart gesteuert werden, dass der Endeffektor 20 zu dem zu transferierenden Nährbodenträger 30 bewegt wird und der Endeffektor 20 den zu transferierenden Nährbodenträger 30 greift.

In einem weiteren Schritt S116 des Verfahrens 300 wird der entnommene Nährbodenträger 30 robotergestützt von der Transferschleuse 14 zu einem freien Halter 40 der Lagereinrichtung 38 transferiert, wobei der Halter 40 als Nährbodenträgerhalter dient. Dazu kann beispielsweise der Roboter 16 beispielsweise derart gesteuert werden, dass der Endeffektor 20 von der Transferschleuse 14 zu dem freien Halter 40 bewegt wird.

In einem weiteren Schritt S117 des Verfahrens 300 wird der transferierte Nährbodenträger 30 robotergestützt in dem feien Halter 40, der als Nährbodenträgerhalter dient, angeordnet. Dabei wird der Nährbodenträger 30 insbesondere auf einer Auflagefläche des Halters 40 abgesetzt. Dazu kann der Roboter 16 beispielsweise derart gesteuert werden, dass der Endeffektor 20 den zu transferierenden Nährbodenträger 30 auf der Auflagefläche aufsetzt und freigibt.

In einem weiteren Schritt S118 des Verfahrens 300 wird bestimmt, ob noch weitere Nährbodenträger 30 in der Transferschleuse 14 bereitgestellt sind. Wenn noch weitere Nährbodenträger 30 in der Transferschleuse 14 bereitgestellt sind, springt das Verfahren 300 zurück zu Schritt S115 und die Schritte S115 bis S118 werden wiederholt. Wenn keine weiteren Nährbodenträger in der Transferschleuse 14 bereitgestellt sind, geht das Verfahren 300 mit Schritt S119 weiter.

Die Schritte S119 und S120 entsprechen den Schritten S65 und S66 des Verfahrens 200 aus Fig. 7.

In einem weiteren Schritt S121 des Verfahrens 300 wird jeweils ein einzelner Nährbodenträger 30 des mindestens einen Nährbodenträgers 30, der noch nicht in der Keimmnonitoringeinrichtung 31 zum Keimmonitoring eingesetzt wurde, robotergestützt aus dem jeweiligen Halter 40 der Lagereinrichtung 38 entnommen. Dazu kann der Roboter 16 beispielsweise derart gesteuert werden, dass der Endeffektor 20 zu dem zu transferierenden Nährbodenträger 30 bewegt wird und der Endeffektor 20 den zu transferierenden Nährbodenträger 30 greift.

In einem weiteren Schritt S122 des Verfahrens 300 wird der entnommene Nährbodenträger 30 robotergestützt von Halter 40 der Lagereinrichtung 38 dem Nährbodenträgerhalter 22 in dem Gehäuse 32 der Keimmonitoringeinrichtung 31 transferiert. Dazu kann beispielsweise der Roboter 16 beispielsweise derart gesteuert werden, dass der Endeffektor 20 von der Halter 40 zu dem Nährbodenträgerhalter 22 bewegt wird.

In einem weiteren Schritt S123 des Verfahrens 300 wird der transferierte Nährbodenträger 30 robotergestützt in dem Nährbodenträgerhalter 22 in dem Gehäuse 32 der Keimmonitoringeinrichtung 31 angeordnet. Dabei wird der Nährbodenträger 30 insbesondere auf einer Auflagefläche des Nährbodenträgerhalters 22 abgesetzt. Dazu kann der Roboter beispielsweise derart gesteuert werden, dass der Endeffektor 20 den zu transferierenden Nährbodenträger 30 auf der Auflagefläche aufsetzt und freigibt.

Die Schritte S124 und S128 entsprechen den Schritten S70 und S74 des Verfahrens 200 aus Fig. 7.

In einem weiteren Schritt S129 des Verfahrens 300 verbleibt dann der Nährbodenträger 30 in geöffneten Zustand eine vordefinierte Zeitspanne in dem Nährbodenträgerhalter 22 der Keimmonitoringeinrichtung, wodurch das Keimmonitoring in dieser Zeitspanne ausgeführt wird. Die vordefinierte Zeitspanne kann beispielsweise 4 Stunden betragen.

Die Schritte S130 und S134 entsprechen den Schritten S75 und S79 des Verfahrens 200 aus Fig. 7.

In einem weiteren Schritt S135 des Verfahrens 300 wird der Nährbodenträger 30 robotergestützt aus dem Nährbodenträgerhalter 22 aus dem Gehäuse 32 der Keimmonitoringeinrichtung 31 entnommen. Dazu kann der Roboter 16 beispielsweise derart gesteuert werden, dass der Endeffektor 18 den geschlossenen Nährbodenträger 30 greift.

In einem weiteren Schritt S136 des Verfahrens 300 wird der entnommene Nährbodenträger 30 robotergestützt von dem Nährbodenträgerhalter 22 in dem Gehäuse 32 der Keimmonitoringeinrichtung 31 zu dem Halter 40 der Lagereinrichtung 38 transferiert. Dazu kann der Roboter 16 beispielsweise derart gesteuert werden, dass der Endeffektor 18 den Nährbodenträger 30 von dem Nährbodenträgerhalter 22 zu der der Transferschleuse 14 bewegt.

In einem weiteren Schritt S137 des Verfahrens 300 wird der transferierte Nährbodenträger 30 robotergestützt in dem Halter 40 der Lagereinrichtung 38 angeordnet. Dabei wird der Nährbodenträger 30 insbesondere auf einer Auflagefläche des Halters 40 abgesetzt. Dazu kann der Roboter 16 beispielsweise derart gesteuert werden, dass der Endeffektor 20 den zu transferierenden Nährbodenträger 30 auf der Auflagefläche aufsetzt und freigibt.

In einem weiteren Schritt S138 des Verfahrens 300 wird bestimmt, ob noch weitere Nährbodenträger 30 in der Lagereinrichtung 38 vorhanden sind, die noch nicht in der Keimmonitoringeinrichtung 31 zum Keimmonitoring eingesetzt wurden. Wenn noch weitere Nährbodenträger 30 in der Lagereinrichtung 38 vorhanden sind, die noch nicht in der Keimmonitoringeinrichtung 31 zum Keimmonitoring eingesetzt wurden, springt das Verfahren 300 zurück zu Schritt S121 und die Schritte S121 bis S138 werden wiederholt. Wenn keine weiteren Nährbodenträger in der Lagereinrichtung 38 vorhanden sind, die noch nicht in der Keimmonitoringeinrichtung 31 zum Keimmonitoring eingesetzt wurden, geht das Verfahren 300 mit Schritt S139 weiter.

Die Schritte S139 und S140 entsprechen den Schritten S83 und S84 des Verfahrens 200 aus Fig. 7.

In einem weiteren Schritt S141 des Verfahrens 300 wird jeder Nährbodenträger 30 einzeln robotergestützt aus dem jeweiligen Halter 40 der Lagereinrichtung 38 entnommen. Dazu kann der Roboter 16 beispielsweise derart gesteuert werden, dass der Endeffektor 18 den geschlossenen Nährbodenträger 30 greift.

In einem weiteren Schritt S142 des Verfahrens 300 wird der entnommene Nährbodenträger 30 robotergestützt von dem Halter 40 der Lagereinrichtung 38 zu der Transferschleuse 14 transferiert. Dazu kann der Roboter 16 beispielsweise derart gesteuert werden, dass der Endeffektor 18 den Nährbodenträger 30 von dem Halter 40 zu der der Transferschleuse 14 bewegt.

In einem weiteren Schritt S143 des Verfahrens 300 wird der transferierte Nährbodenträger 30 robotergestützt in der Transferschleuse 14 angeordnet. Dazu kann der Roboter 16 beispielsweise derart gesteuert werden, dass der Endeffektor 18 den Nährbodenträger 30 in der Transferschleuse 14 ablegt und freigibt.

In einem weiteren Schritt S144 des Verfahrens 300 wird bestimmt, ob noch weitere Nährbodenträger 30 in der Lagereinrichtung 38 auf einem Halter 40 angeordnet sind. Wenn noch weitere Nährbodenträger 30 in der Lagereinrichtung 38 auf einem Halter 40 angeordnet sind, springt das Verfahren 300 zurück zu Schritt S141 und die Schritte S141 bis S143 werden wiederholt. Wenn keine weiteren Nährbodenträger in der Lagereinrichtung 38 auf einem Halter 40 angeordnet sind und somit alle Nährbodenträger in der Transferschleuse angeordnet sind, ist das Verfahren beendet.

## Patentansprüche

1. Verfahren (100, 200, 300) zum automatisierten Keimmonitoring in einem Isolator (12), wobei der Isolator (12) eine Transferschleuse (14) aufweist, wobei das Verfahren (100, 200, 300) die folgenden Schritte aufweist:
- erstes Bereitstellen mindestens eines Nährbodenträgerhalters (22) an jeweils einer ersten Position (46) innerhalb des Isolators (12);
- zweites Bereitstellen mindestens eines Nährbodenträgers (30) innerhalb der Transferschleuse (14);
- erstes robotergestütztes Transferieren jeweils eines einzelnen Nährbodenträgers (30) des mindestens einen Nährbodenträgers (30) von der Transferschleuse (14) zu einem freien Nährbodenträgerhalter (22) des mindestens einen Nährbodenträgerhalters (22); und
- erstes robotergestütztes Anordnen des transferierten Nährbodenträgers (30) in dem freien Nährbodenträgerhalter (22).

2. Verfahren (100, 200, 300) nach Anspruch 1, wobei das Verfahren (100, 200, 300) vor dem Schritt des ersten robotergestützten Transferierens den folgenden Schritt aufweist:
- erstes robotergestütztes Entnehmen des zu transferierenden Nährbodenträgers (30) aus der Transferschleuse (14).

3. Verfahren (100, 200, 300) nach Anspruch 1 oder 2, wobei jeder Nährbodenträger eine Schale (42) mit Nährboden und einen Deckel (44) aufweist, der auf einer Öffnung der Schale (42) aufgesetzt ist, wobei das Verfahren (100, 200, 300) des Weiteren die folgenden Schritte aufweist:
- drittes Bereitstellen mindestens eines Deckelhalters (24) zur Ablage des Deckels (44) des transferierten Nährbodenträgers (30), wobei der mindestens eine Deckelhalter (24) an jeweils einer zweiten Position (48) innerhalb des Isolators (12) bereitgestellt wird.

4. Verfahren (100, 200, 300) nach Anspruch 3, wobei das Verfahren (100, 200, 300) des Weiteren die folgenden Schritte aufweist:
- robotergestütztes Abnehmen des Deckels (44) von der Schale (42) des transferierten Nährbodenträgers (30);
- zweites robotergestütztes Transferieren des Deckels (44) von dem Nährbodenträgerhalter (22) zu einem freien Deckelhalter (24) des mindestens einen Deckelhalters (24); und
- robotergestütztes Ablegen des Deckels (44) auf dem freien Deckelhalter (24);
insbesondere wobei das Verfahren (100, 200, 300) des Weiteren die folgenden Schritte aufweist, nachdem der transferierte Nährbodenträger (30) eine vordefinierte Zeitspanne in dem entsprechenden Nährbodenträgerhalter (22) verbracht hat:
- robotergestütztes Aufnehmen des Deckels (44) von dem Deckelhalter (24);
- drittes robotergestütztes Transferieren des Deckels (44) von dem Deckelhalter (24) zu dem entsprechenden Nährbodenträgerhalter (22); und
- robotergestütztes Aufsetzen des Deckels (44) auf die Schale (42) des Nährbodenträgers (30).

5. Verfahren (100, 200, 300) nach einem der Ansprüche 1 bis 4, wobei das Verfahren (100, 200, 300) des Weiteren die folgenden Schritte aufweist, nachdem der transferierte Nährbodenträger (30) eine vordefinierte Zeitspanne in dem entsprechenden Nährbodenträgerhalter (22) verbracht hat:
- zweites robotergestütztes Entnehmen des Nährbodenträgers (30) aus dem Nährbodenträgerhalter (22);
- viertes robotergestütztes Transferieren des Nährbodenträgers (30) von dem Nährbodenträgerhalter (22) zu der Transferschleuse (14); und
- zweites robotergestütztes Anordnen des Nährbodenträgers (30) in der Transferschleuse (14).

6. Verfahren (100, 200, 300) nach einem der Ansprüche 1 bis 5, wobei in dem Schritt des ersten Bereitstellens eine Keimmonitoringeinrichtung (31) mit zumindest einem Nährbodenträgerhalter (22) bereitgestellt wird, wobei die Keimmonitoringeinrichtung (31) ein Gehäuse (32) aufweist, in dem der zumindest eine Nährbodenträgerhalter (22) angeordnet ist, wobei das Gehäuse (32) einen Gehäusedeckel (34) aufweist, wobei das Verfahren (100, 200, 300) des Weiteren die folgenden Schritte aufweist:
- viertes Bereitstellen eines Gehäusedeckelhalters (36) innerhalb des Isolators (12) an einer dritten Position (50);
- robotergestütztes Abnehmen des Gehäusedeckels (34); und
- robotergestütztes Ablegen des Gehäusedeckels (34) auf dem Gehäusedeckelhalter (36).

7. Verfahren (100, 200, 300) nach Anspruch 6, wobei in dem Schritt des zweiten Bereitstellens eine Mehrzahl von Nährbodenträgern (30) in der Transferschleuse (14) bereitgestellt wird, wobei in dem Schritt des dritten Bereitstellens eine Lagereinrichtung (38) mit einer Mehrzahl von Haltern (40) an der zweiten Position (48) bereitgestellt wird, wobei die Halter (40) jeweils als Nährbodenträgerhalter (22) oder Deckelhalter (24) dienen können, insbesondere wobei in dem Schritt des zweiten Bereitstellens die Mehrzahl von Nährbodenträgern (30) in einer Nährbodenträgerhalteeinrichtung in der Transferschleuse (14) bereitgestellt wird.

8. Verfahren (100, 200, 300) nach einem der Ansprüche 1 bis 7, wobei in dem Schritt des zweiten Bereitstellens jeder Nährbodenträger (30) in einem weiteren Nährbodenträgerhalter bereitgestellt wird, wobei jeder weitere Nährbodenträgerhalter in der Transferschleuse (14) angeordnet wird, insbesondere wobei die Nährbodenträgerhalteeinrichtung jeden weiteren Nährbodenträgerhalter aufweist, insbesondere wobei sich jeder weitere Nährbodenträgerhalter zumindest teilweise aus der Transferschleuse (14) heraus in den Isolator (10) erstrecken kann, insbesondere wobei der Schritt des ersten robotergestützten Transferierens derart erfolgt, dass sich der jeweilige weitere Nährbodenträgerhalter zumindest teilweise aus der Transferschleuse (14) heraus in den Isolator (10) erstreckt und der entsprechende Nährbodenträger (30) robotergestützt von dem jeweiligen weiteren Nährbodenträgerhalter zu dem freien Nährbodenträgerhalter (22) innerhalb des Isolators (10) transferiert wird.

9. Verfahren (100, 200, 300) nach einem der Ansprüche 1 bis 8, wobei in dem Isolator (12) ein Roboter (16) angeordnet ist, wobei der Roboter (16) einen Endeffektor (20) zum Handhaben eines Nährbodenträgers (30) und eine Trägerstruktur (18) zum Tragen des Endeffektors (20) aufweist, wobei die Trägerstruktur (18) dazu ausgebildet ist, den Endeffektor (20) im Isolator (12) zu bewegen, wobei der Endeffektor (20) dazu ausgebildet ist, den Nährbodenträger (30) zu greifen.

10. Verfahren (100, 200, 300) nach einem der Ansprüche 1 bis 9, wobei die Anzahl der Nährbodenträgerhalter (22), die in dem Isolator (12) bereitgestellt werden, gleich oder größer der Anzahl der Nährbodenträger (30) ist, die in der Transferschleuse (14) bereitgestellt werden, und/oder , wobei die Anzahl der Deckelhalter (24), die in dem Isolator (12) bereitgestellt werden, gleich oder größer der Anzahl der Nährbodenträger (30) ist, die in der Transferschleuse (14) bereitgestellt werden.

11. System (10) zum automatisierten Keimmonitoring in einem Isolator (12), wobei das System (10) den Isolator (12), mindestens einen Nährbodenträgerhalter (22), einen in dem Isolator (12) angeordneten Roboter (16) und eine Steuerungseinrichtung (26) aufweist, wobei der Isolator (12) eine Transferschleuse (14) aufweist, wobei in der Transferschleuse (14) mindestens ein Nährbodenträger (30) bereitgestellt ist, wobei der Nährbodenträgerhalter (22) innerhalb des Isolators an einer ersten Position (46) angeordnet ist, wobei der Roboter (16) einen Endeffektor (20) zum Handhaben eines Nährbodenträgers (30) und eine Trägerstruktur (18) zum Tragen des Endeffektors (20) aufweist, wobei die Trägerstruktur (18) dazu ausgebildet ist, den Endeffektor (20) im Isolator (12) zu bewegen, wobei der Endeffektor (20) dazu ausgebildet ist, den Nährbodenträger (30) zu greifen, wobei die Steuerungseinrichtung (26) dazu eingerichtet ist, die folgenden Schritte auszuführen:
- erstes robotergestütztes Transferieren jeweils eines einzelnen Nährbodenträgers (30) des mindestens einen Nährbodenträgers (30) von der Transferschleuse (14) zu einem freien Nährbodenträgerhalter (22) des mindestens einen Nährbodenträgerhalters (22); und
- erstes robotergestütztes Anordnen des transferierten Nährbodenträgers (30) in dem freien Nährbodenträgerhalter (22).

12. System (10) nach Anspruch 11, wobei der Endeffektor (20) eine Aufnahme (66) zum Aufnehmen des Nährbodenträger (30) aufweist, die zwischen einer Aufnahmestellung, in der der Nährbodenträger (30) aufgenommen werden kann, und einer Greifstellung, in der der Nährbodenträger (30) gegriffen werden kann, verlagerbar ist, insbesondere wobei jeder Nährbodenträger (30) zum Transferieren jeweils mittels des Endeffektors (20) gegriffen und mittels des Roboters (16) im Isolator (12) bewegt werden kann.

13. System (10) nach Anspruch 11 oder 12, wobei das System (10) eine Keimmonitoringeinrichtung (31) mit zumindest einem Nährbodenträgerhaltern (22) aufweist, insbesondere wobei die Keimmonitoringeinrichtung (31) ein Gehäuse (32) aufweist, in der zumindest eine Nährbodenträgerhalter (30) angeordnet ist, wobei das Gehäuse (32) einen Gehäusedeckel (34) aufweist, wobei der Gehäusedeckel (34) mittels des Roboters (16) abnehmbar ist, wobei das System (10) einen Gehäusedeckelhalter (36) zur Ablage des Gehäusedeckels (34) aufweist, wobei der Gehäusedeckelhalter (36) innerhalb des Isolators (12) an einer dritten Position (50) angeordnet ist.

14. System (10) nach einem der Ansprüche 11 bis 13, wobei jeder Nährbodenträger (30) eine Schale (42) mit Nährboden und einen Deckel (44) aufweist, der auf einer Öffnung der Schale (42) aufgesetzt ist, wobei der Deckel (44) mittels des Roboters (16) abnehmbar ist, wobei das System (10) des Weiteren mindestens einen Deckelhalter (24) zur Ablage des Deckels (44) aufweist, wobei der mindestens eine Deckelhalter (24) innerhalb des Isolator (12) an jeweils einer zweiten Position (48) angeordnet ist.

15. Computerprogramm mit einem Programmcode, der dazu ausgebildet ist, wenn er in der Steuerungseinrichtung (26) des Systems (10) nach einem der Ansprüche 11 bis 14 ausgeführt wird, die folgenden Schritte auszuführen:
- erstes robotergestütztes Transferieren jeweils eines einzelnen Nährbodenträgers (30) des mindestens einen Nährbodenträgers (30) von der Transferschleuse (14) zu einem freien Nährbodenträgerhalter (22) des mindestens einen Nährbodenträgerhalters (22); und
- erstes robotergestütztes Anordnen des transferierten Nährbodenträgers (30) in dem freien Nährbodenträgerhalter (22).

## Claims

1. A method (100, 200, 300) for automated microbial monitoring in an isolator (12), the isolator (12) having a transfer lock (14), the method (100, 200, 300) comprising the following steps:
- first providing of at least one nutrient medium carrier holder (22) at in each case a first position (46) within the isolator (12);
- second providing of at least one nutrient medium carrier (30) within the transfer lock (14);
- first robot-assisted transferring of an individual nutrient medium carrier (30) of the at least one nutrient medium carrier (30) from the transfer lock (14) to a free nutrient medium carrier holder (22) of the at least one nutrient medium carrier holder (22); and
- first robot-assisted placing of the transferred nutrient medium carrier (30) in the free nutrient medium carrier holder (22).

2. The method (100, 200, 300) as claimed in claim 1, wherein the method (100, 200, 300) comprises, before the step of the first robot-assisted transferring, the following step:
- first robot-assisted removing of the nutrient medium carrier (30), which is to be transferred, from the transfer lock (14).

3. The method (100, 200, 300) as claimed in claim 1 or 2, wherein each nutrient medium carrier has a dish (42) with nutrient medium and a lid (44) which is placed on an opening of the dish (42), the method (100, 200, 300) further comprising the following steps:
- third providing of at least one lid holder (24) for storing the lid (44) of the transferred nutrient medium carrier (30), the at least one lid holder (24) being provided at in each case a second position (48) within the isolator (12).

4. The method (100, 200, 300) as claimed in claim 3, wherein the method (100, 200, 300) further comprises the following steps:
- robot-assisted removing of the lid (44) from the dish (42) of the transferred nutrient medium carrier (30);
- second robot-assisted transferring of the lid (44) from the nutrient medium carrier holder (22) to a free lid holder (24) of the at least one lid holder (24); and
- robot-assisted depositing of the lid (44) on the free lid holder (24).
in particular wherein the method (100, 200, 300) further comprises the following steps, after the transferred nutrient medium carrier (30) has spent a predefined period of time in the corresponding nutrient medium carrier holder (22):
- robot-assisted picking-up of the lid (44) from the lid holder (24);
- third robot-assisted transferring of the lid (44) from the lid holder (24) to the corresponding nutrient medium carrier holder (22); and
- robot-assisted fitting of the lid (44) onto the dish (42) of the nutrient medium carrier (30).

5. The method (100, 200, 300) as claimed in one of claims 1 to 4, wherein the method (100, 200, 300) further comprises the following steps, after the transferred nutrient medium carrier (30) has spent a predefined period of time in the corresponding nutrient medium carrier holder (22):
- second robot-assisted removing of the nutrient medium carrier (30) from the nutrient medium carrier holder (22);
- fourth robot-assisted transferring of the nutrient medium carrier (30) from the nutrient medium carrier holder (22) to the transfer lock (14); and
- second robot-assisted placing of the nutrient medium carrier (30) in the transfer lock (14).

6. The method (100, 200, 300) as claimed in one of claims 1 to 5, wherein, in the step of the first providing, a microbial monitoring device (31) with at least one nutrient medium carrier holder (22) is provided, wherein the microbial monitoring device (31) has a housing (32) in which the at least one nutrient medium carrier holder (22) is arranged, the housing (32) having a housing lid (34), wherein the method (100, 200, 300) further comprises the following steps:
- fourth providing of a housing lid holder (36) within the isolator (12) at a third position (50);
- robot-assisted removing of the housing lid (34); and
- robot-assisted depositing of the housing lid (34) on the housing lid holder (36).

7. The method (100, 200, 300) as claimed in claim 6, wherein, in the step of the second providing, a plurality of nutrient medium carriers (30) are provided in the transfer lock (14), wherein, in the step of the third providing, a storage device (38) with a plurality of holders (40) is provided at the second position (48), wherein the holders (40) are each able to serve as a nutrient medium carrier holder (22) or lid holder (24), in particular wherein, in the step of the second providing, the plurality of nutrient medium carriers (30) are provided in a nutrient medium carrier retainer in the transfer lock (14).

8. The method (100, 200, 300) as claimed in one of claims 1 to 7, wherein, in the step of the second providing, each nutrient medium carrier (30) is provided in a further nutrient medium carrier holder, wherein each further nutrient medium carrier holder is arranged in the transfer lock (14), in particular wherein the nutrient medium carrier retainer has each further nutrient medium carrier holder, in particular wherein each further nutrient medium carrier holder can extend at least partially out of the transfer lock (14) into the isolator (10), in particular wherein the step of the first robot-assisted transferring takes place in such a way that the respective further nutrient medium carrier holder extends at least partially out of the transfer lock (14) into the isolator (10) and the corresponding nutrient medium carrier (30) is transferred, with robot assistance, from the respective further nutrient medium carrier holder to the free nutrient medium carrier holder (22) within the isolator (10).

9. The method (100, 200, 300) as claimed in one of claims 1 to 8, wherein a robot (16) is arranged in the isolator (12), wherein the robot (16) has an end effector (20) for handling a nutrient medium carrier (30) and a support structure (18) for supporting the end effector (20), wherein the support structure (18) is configured to move the end effector (20) in the isolator (12), wherein the end effector (20) is configured to grip the nutrient medium carrier (30).

10. The method (100, 200, 300) as claimed in one of claims 1 to 9, wherein the number of nutrient medium carrier holders (22) provided in the isolator (12) is equal to or greater than the number of nutrient medium carriers (30) provided in the transfer lock (14), and/or , wherein the number of lid holders (24) provided in the isolator (12) is equal to or greater than the number of nutrient medium carriers (30) provided in the transfer lock (14).

11. A system (10) for automated microbial monitoring in an isolator (12), wherein the system (10) has the isolator (12), at least one nutrient medium carrier holder (22), a robot (16) arranged in the isolator (12), and a control device (26), wherein the isolator (12) has a transfer lock (14), wherein at least one nutrient medium carrier (30) is provided in the transfer lock (14), wherein the nutrient medium carrier holder (22) is arranged at a first position (46) within the isolator, wherein the robot (16) has an end effector (20) for handling a nutrient medium carrier (30) and a support structure (18) for supporting the end effector (20), wherein the support structure (18) is configured to move the end effector (20) in the isolator (12), wherein the end effector (20) is configured to grip the nutrient medium carrier (30), and wherein the control device (26) is configured to carry out the following steps:
- first robot-assisted transferring of in each case an individual nutrient medium carrier (30) of the at least one nutrient medium carrier (30) from the transfer lock (14) to a free nutrient medium carrier holder (22) of the at least one nutrient medium carrier holder (22); and
- first robot-assisted placing of the transferred nutrient medium carrier (30) in the free nutrient medium carrier holder (22).

12. The system (10) as claimed in claim 11, wherein the end effector (20) has a receptacle (66) for receiving the nutrient medium carrier (30), which receptacle (66) is movable between a receiving position, in which the nutrient medium carrier (30) can be received, and a gripping position, in which the nutrient medium carrier (30) can be gripped, in particular wherein each nutrient medium carrier (30) for transferring can be gripped by means of the end effector (20) and moved by means of the robot (16) within the isolator (12).

13. The system (10) as claimed in claim 11 or 12, wherein the system (10) has a microbial monitoring device (31) with at least one nutrient medium carrier holder (22), in particular wherein the microbial monitoring device (31) has a housing (32) in which at least one nutrient medium carrier holder (30) is arranged, wherein the housing (32) has a housing lid (34), wherein the housing lid (34) is removable by means of the robot (16), wherein the system (10) has a housing lid holder (36) for storing the housing lid (34), wherein the housing lid holder (36) is arranged at a third position (50) within the isolator (12).

14. The system (10) as claimed in one of claims 11 to 13, wherein each nutrient medium carrier (30) has a dish (42) with nutrient medium and a lid (44) which is placed on an opening of the dish (42), wherein the lid (44) can be removed by means of the robot (16), wherein the system (10) furthermore has at least one lid holder (24) for storing the lid (44), wherein the at least one lid holder (24) is arranged inside the isolator (12) at a second position (48) in each case.

15. A computer program with a program code which is configured, when executed in the control device (26) of the system (10) as claimed in one of claims 11 to 14, to carry out the following steps:
- first robot-assisted transferring of an individual nutrient medium carrier (30) of the at least one nutrient medium carrier (30) from the transfer lock (14) to a free nutrient medium carrier holder (22) of the at least one nutrient medium carrier holder (22); and
- first robot-assisted placing of the transferred nutrient medium carrier (30) in the free nutrient medium carrier holder (22).

## Revendications

1. Procédé (100, 200, 300) pour la surveillance automatisée de microbes dans un isolateur (12), dans lequel l'isolateur (12) présente un sas de transfert (14), dans lequel le procédé (100, 200, 300) présente les étapes suivantes :
- première mise à disposition d'au moins un moyen de maintien de support de milieu de culture (22) à respectivement une première position (46) à l'intérieur de l'isolateur (12) ;
- deuxième mise à disposition d'au moins un support de milieu de culture (30) à l'intérieur du sas de transfert (14) ;
- premier transfert assisté par robot de respectivement un seul support de milieu de culture (30) de l'au moins un support de milieu de culture (30) depuis le sas de transfert (14) vers un moyen de maintien de support de milieu de culture (22) libre de l'au moins un moyen de maintien de support de milieu de culture (22) ; et
- première disposition assistée par robot du support de milieu de culture (30) transféré dans le moyen de maintien de support de milieu de culture (22) libre.

2. Procédé (100, 200, 300) selon la revendication 1, dans lequel le procédé (100, 200, 300) présente l'étape suivante avant l'étape de premier transfert assisté par robot :
- premier prélèvement assisté par robot du support de milieu de culture (30) à transférer hors du sas de transfert (14).

3. Procédé (100, 200, 300) selon la revendication 1 ou 2, dans lequel chaque support de milieu de culture présente une boîte (42) comportant un milieu de culture et un couvercle (44) placé sur une ouverture de la boîte (42), dans lequel le procédé (100, 200, 300) présente en outre les étapes suivantes :
- troisième mise à disposition d'au moins un moyen de maintien de couvercle (24) pour déposer le couvercle (44) du support de milieu de culture (30) transféré, dans lequel l'au moins un moyen de maintien de couvercle (24) est mis à disposition à respectivement une deuxième position (48) à l'intérieur de l'isolateur (12).

4. Procédé (100, 200, 300) selon la revendication 3, dans lequel le procédé (100, 200, 300) présente en outre les étapes suivantes :
- retrait assisté par robot du couvercle (44) de la boîte (42) du support de milieu de culture (30) transféré ;
- deuxième transfert assisté par robot du couvercle (44) depuis le moyen de maintien de support de milieu de culture (22) vers un moyen de maintien de couvercle (24) libre de l'au moins un moyen de maintien de couvercle (24) ; et
- dépose assistée par robot du couvercle (44) sur le moyen de maintien de couvercle (24) libre ;
en particulier, dans lequel le procédé (100, 200, 300) présente en outre les étapes suivantes après que le support de milieu de culture (30) transféré a passé une période de temps prédéfinie dans le moyen de maintien de support de milieu de culture (22) correspondant :
- réception assistée par robot du couvercle (44) par le moyen de maintien de couvercle (24) ;
- troisième transfert assisté par robot du couvercle (44) depuis le moyen de maintien de couvercle (24) vers le moyen de maintien de support de milieu de culture (22) correspondant ; et
- mise en place assistée par robot du couvercle (44) sur la boîte (42) du support de milieu de culture (30).

5. Procédé (100, 200, 300) selon l'une des revendications 1 à 4, dans lequel le procédé (100, 200, 300) présente en outre les étapes suivantes après que le support de milieu de culture (30) transféré a passé une période de temps prédéfinie dans le moyen de maintien de support de milieu de culture (22) correspondant :
- deuxième retrait assisté par robot du support de milieu de culture (30) du moyen de maintien de support de milieu de culture (22) ;
- quatrième transfert assisté par robot du support de milieu de culture (30) depuis le moyen de maintien de support de milieu de culture (22) vers le sas de transfert (14) ; et
- seconde disposition assistée par robot du support de milieu de culture (30) dans le sas de transfert (14).

6. Procédé (100, 200, 300) selon l'une des revendications 1 à 5, dans lequel, à l'étape de première mise à disposition, un dispositif de surveillance de microbes (31) est mis à disposition avec au moins un moyen de maintien de support de milieu de culture (22), dans lequel le dispositif de surveillance de microbes (31) présente un boîtier (32) dans lequel l'au moins un moyen de maintien de support de milieu de culture (22) est disposé, dans lequel le boîtier (32) présente un couvercle de boîtier (34), dans lequel le procédé (100, 200, 300) présente en outre les étapes suivantes :
- quatrième mise à disposition d'un moyen de maintien de couvercle de boîtier (36) à l'intérieur de l'isolateur (12) à une troisième position (50) ;
- retrait assisté par robot du couvercle du boîtier (34) ; et
- dépose assistée par robot du couvercle de boîtier (34) sur le moyen de maintien de couvercle de boîtier (36).

7. Procédé (100, 200, 300) selon la revendication 6, dans lequel, à l'étape de deuxième mise à disposition, une pluralité de supports de milieu de culture (30) est mise à disposition dans le sas de transfert (14), dans lequel, à l'étape de troisième mise à disposition, un dispositif de stockage (38) comportant une pluralité de moyens de maintien (40) est mis à disposition à la deuxième position (48), dans lequel les moyens de maintien (40) peuvent servir respectivement de moyen de maintien de support de milieu de culture (22) ou de moyen de maintien de couvercle (24), en particulier dans lequel, à l'étape de deuxième mise à disposition, la pluralité de supports de milieu de culture (30) est mise à disposition dans un dispositif de maintien de support de milieu de culture dans le sas de transfert (14).

8. Procédé (100, 200, 300) selon l'une des revendications 1 à 7, dans lequel, à l'étape de deuxième mise à disposition, chaque support de milieu de culture (30) est mis à disposition dans un autre moyen de maintien de support de milieu de culture, dans lequel chaque autre moyen de maintien de support de milieu de culture est disposé dans le sas de transfert (14), en particulier dans lequel le dispositif de maintien de support de milieu de culture présente chaque autre moyen de maintien de support de milieu de culture, en particulier dans lequel chaque autre moyen de maintien de support de milieu de culture peut s'étendre au moins partiellement hors du sas de transfert (14) dans l'isolateur (10), en particulier dans lequel l'étape de premier transfert assisté par robot est effectuée de telle sorte que l'autre moyen de maintien de support de milieu de culture respectif s'étend au moins partiellement hors du sas de transfert (14) dans l'isolateur (10) et le support de milieu de culture (30) correspondant est transféré de manière assistée par robot depuis l'autre moyen de maintien de support de milieu de culture respectif jusqu'au moyen de maintien de support de milieu de culture (22) libre à l'intérieur de l'isolateur (10).

9. Procédé (100, 200, 300) selon l'une des revendications 1 à 8, dans lequel un robot (16) est disposé dans l'isolateur (12), dans lequel le robot (16) présente un effecteur terminal (20) permettant de manipuler un support de milieu de culture (30) et une structure de support (18) permettant de supporter l'effecteur terminal (20), dans lequel la structure de support (18) est conçue pour déplacer l'effecteur terminal (20) dans l'isolateur (12), dans lequel l'effecteur terminal (20) est conçu pour saisir le support de milieu de culture (30).

10. Procédé (100, 200, 300) selon l'une des revendications 1 à 9, dans lequel le nombre de moyens de maintien de support de milieu de culture (22) mis à disposition dans l'isolateur (12) est égal ou supérieur au nombre de supports de milieu de culture (30) mis à disposition dans le sas de transfert (14), et/ou, dans lequel le nombre de moyens de maintien de couvercle (24) mis à disposition dans l'isolateur (12) est égal ou supérieur au nombre de supports de milieu de culture (30) mis à disposition dans le sas de transfert (14).

11. Système (10) pour la surveillance automatisée de microbes dans un isolateur (12), dans lequel le système (10) présente l'isolateur (12), au moins un moyen de maintien de support de milieu de culture (22), un robot (16) disposé dans l'isolateur (12) et un dispositif de commande (26), dans lequel l'isolateur (12) présente un sas de transfert (14), dans lequel au moins un support de milieu de culture (30) est mis à disposition dans le sas de transfert (14), dans lequel le moyen de maintien de support de milieu de culture (22) est disposé à l'intérieur de l'isolateur à une première position (46), dans lequel le robot (16) présente un effecteur terminal (20) permettant de manipuler un support de milieu de culture (30) et une structure de support (18) permettant de supporter l'effecteur terminal (20), dans lequel la structure de support (18) est conçue pour déplacer l'effecteur terminal (20) dans l'isolateur (12), dans lequel l'effecteur terminal (20) est conçu pour saisir le support de milieu de culture (30), dans lequel le dispositif de commande (26) est configuré pour exécuter les étapes suivantes :
- premier transfert assisté par robot de respectivement un seul support de milieu de culture (30) de l'au moins un support de milieu de culture (30) depuis le sas de transfert (14) vers un moyen de maintien de support de milieu de culture (22) libre de l'au moins un moyen de maintien de support de milieu de culture (22) ; et
- première disposition assistée par robot du support de milieu de culture (30) transféré dans le moyen de maintien de support de milieu de culture (22) libre.

12. Système (10) selon la revendication 11, dans lequel l'effecteur terminal (20) présente un réceptacle (66) permettant de recevoir le support de milieu de culture (30) et pouvant être déplacé entre une position de réception dans laquelle le support de milieu de culture (30) peut être reçu, et une position de préhension dans laquelle le support de milieu de culture (30) peut être saisi, en particulier dans lequel chaque support de milieu de culture (30) peut être saisi pour être transféré respectivement au moyen de l'effecteur terminal (20) et peut être déplacé au moyen du robot (16) dans l'isolateur (12).

13. Système (10) selon la revendication 11 ou 12, dans lequel le système (10) présente un dispositif de surveillance de microbes (31) comportant au moins un moyen de maintien de support de milieu de culture (22), en particulier dans lequel le dispositif de surveillance de microbes (31) présente un boîtier (32) dans lequel est disposé au moins un moyen de maintien de support de milieu de culture (30), dans lequel le boîtier (32) présente un couvercle de boîtier (34), dans lequel le couvercle de boîtier (34) peut être retiré au moyen du robot (16), dans lequel le système (10) présente un moyen de maintien de couvercle de boîtier (36) permettant de déposer le couvercle de boîtier (34), dans lequel le moyen de maintien de couvercle de boîtier (36) est disposé à l'intérieur de l'isolateur (12) à une troisième position (50).

14. Système (10) selon l'une des revendications 11 à 13, dans lequel chaque support de milieu de culture (30) présente une boîte (42) comportant un milieu de culture et un couvercle (44) placé sur une ouverture de la boîte (42), dans lequel le couvercle (44) peut être retiré au moyen du robot (16), dans lequel le système (10) présente en outre au moins un moyen de maintien de couvercle (24) permettant de déposer le couvercle (44), dans lequel l'au moins un moyen de maintien de couvercle (24) est disposé à l'intérieur de l'isolateur (12) à respectivement une deuxième position (48).

15. Programme informatique comportant un code de programme qui est configuré pour, lorsqu'il est exécuté dans le dispositif de commande (26) du système (10) selon l'une des revendications 11 à 14, exécuter les étapes suivantes :
- premier transfert assisté par robot de respectivement un seul support de milieu de culture (30) de l'au moins un support de milieu de culture (30) depuis le sas de transfert (14) vers un moyen de maintien de support de milieu de culture (22) libre de l'au moins un moyen de maintien de support de milieu de culture (22) ; et
- première disposition assistée par robot du support de milieu de culture (30) transféré dans le moyen de maintien de support de milieu de culture (22) libre.
